# EUROPEAN PATENT SPECIFICATION

(11) **EP 4 341 239 B1**
(45) Date of publication and mention of the grant of the patent: **19.08.2026**
(21) Application number: 22727933.8
(22) Date of filing: 10.05.2022
(51) Int. Cl.: C07C 201/12, C07C 253/30, C07C 205/06, C07C 205/35, C07C 255/50, C07C 205/57

(54) **PROCESS FOR PREPARING SUBSTITUTED BIPHENYLS VIA SUZUKI COUPLING OF ARYL-CHLORIDES**
VERFAHREN ZUR HERSTELLUNG SUBSTITUIERTER BIPHENYLE ÜBER SUZUKI-KUPPLUNG VON ARYLCHLORIDEN
PROCÉDÉ DE PRÉPARATION DE BIPHÉNYLES SUBSTITUÉS PAR COUPLAGE DE SUZUKI DE CHLORURES D'ARYLE

(30) Priority: 19.05.2021 EP 21174585
(43) Date of publication of application: 27.03.2024
(73) Proprietor: BASF Agricultural Solutions Deutschland GmbH, 67117 Limburgerhof (DE)
(72) Inventor: SCHAUB, Thomas, 67056 Ludwigshafen (DE); PETKOVA, Desislava Slavcheva, 67056 Ludwigshafen (DE); DIETZ, Jochen, 67056 Ludwigshafen (DE); GOETZ, Roland, 67056 Ludwigshafen (DE); HASHMI, A. Stephen K., 69117 Heidelberg (DE); ORECCHIA, Patrizio, 69120 Heidelberg (DE)
(74) Representative: BASF IP Association
(86) International application number: PCT/EP2022/062558
(87) International publication number: WO 2022/243099

(56) References cited:
- WO-A1-2006/092429

## Description

The present invention relates to a process for preparing substituted biphenyls via Suzuki coupling of aryl-chlorides using a palladium catalyst with specific phosphorus ligands in water as solvent without the use of surfactants or phase transfer catalyst
Functionalized biphenyl compounds are of great interest especially as pharmaceuticals
and pesticides, and as precursors of such active ingredients. For instance, 2-nitro and 2-aminobiphenyls are important precursors for aryl- and heteroarylcarboxamides which find use as fungicides, and for which boscalid^{®}, fluxapyroxad, bixafen or pyraziflumid are prominent representatives. For their synthesis, a series of organometallic methods is available, which offer efficient access to a multitude of biphenyl derivatives. The most frequently applied is the Suzuki coupling.

The Suzuki coupling (also called Suzuki-Miyaura coupling or Suzuki reaction or Suzuki-Miyaura reaction) is a cross coupling reaction in which an organoboron compound is reacted with an organic halogenide or sulfonate in the presence of a transition metal catalyst, mostly a Pd or Ni catalyst, and in general also of a base.

Principally, the known processes for preparing nitro- or aminobiphenyls via Suzuki coupling work well, at least on a laboratory scale. However, there is still room for improvement, especially with respect to an application in large-scale industrial processes. For instance, the amount of required Pd in the catalyst is still rather high and minimizing the amount of organic solvents is for economic as well as ecologic reasons desired.

WO 2006/092429 discloses a process for producing substituted biphenyls by reacting a phenyl halogenide with a diphenylborinic acid in the presence inter alia of an organic solvent, preferably in a mixture of tetrahydrofurane and water.

WO 2015/011032 relates to a process for preparing chlorinated biphenylanilines or anilides by Suzuki coupling using a palladium catalyst containing an optionally substituted di-tert-butylphenyl phosphine or a salt thereof as ligand. This catalyst is said to avoid the undesired formation of triphenyl compounds. In the halide starting compound II the leaving group Hal is Br or I. In the examples the coupling reaction is carried out in a mixture of water and 1-butanol as solvent in the presence of potassium carbonate as base. The Pd catalyst is used in an amount of 0.12 mol%, calculated on the basis of the Pd content and relative to 1 mol of the halide.

Although in this process the amount of Pd is already reduced as compared to older processes, there is still room for improvement. Moreover, the use of aromatic bromides or iodides is not desirable, not only because of their cost, especially in case of the iodide, but also because of environmental concerns connected with bromide or iodide containing wastewater.

WO 2018/149813 A1 relates to a process for preparing substituted 2-nitrobiphenyls via Suzuki coupling using a palladium catalyst with specific phosphorus ligands containing at least one aryl substituent and a solvent mixture containing water and an organic solvent which is at least partially miscible with water.

Although in this process the preferred aryl-chlorides are used as coupling partner, still significant amounts (at least 20 % by weight in the reaction mixture) of a water miscible organic solvent like tetrahydrofurane is necessary to carry out the reaction. The water soluble organic solvent required for the reaction needs to be separated from the reaction mixture after the reaction, for example by distillation, in order that it do not end up in the waste water, which requires an additional energy consuming step before further product isolation. In RSC Advances, 2011, 1, 1013-1019 is a system presented, which allows the Suzuki coupling of aryl-chlorides in water at low palladium-loadings. The catalyst is palladium immobilized on Agarose. A drawback of this system is that especially the yields in case of the relevant substituted aryl-chloride as para-Nitro-chlorobenzene only gives a moderate yield of 70% of the coupling product.

In Chinese Journal of Chemistry, 2015, 33, 705-710 a surfactant connected palladium-catalyst is used with water as the sole solvent. But also in this case, for the relevant substituted aryl-chloride as para-nitro-chlorobenzene, high catalyst loadings of 0.05 mol% are necessary and also 0.5 equivalents of tetrabutylammoniumbromide as an additive besides K₂CO₃. For an economic process, it would be desirable to use a low as possible palladium-loading and also to reduce the number of additives in the system.

In Organometallics 2014, 33, 6544-6549, a Pd-catalyst with N-heterocyclic carbenes as ligands are used for the Suzuki-coupling of aryl-chlorides using water as the sole solvent. But also, relatively high palladium-loadings of 0.1 mol% are necessary for the coupling of nitro-substituted chlorobenzene and the coupling product is only obtained in 31% yield.

In Journal of the American Chemical Society, 2012, 134, 3190-3198, a polymeric imidazole palladium catalyst is used in the Suzuki-coupling of aryl-chlorides in water as the solvent. But in this system, in addition to the necessary base, also at least one equivalent of tetrabutylammoni-umfluoride is required, to achieve high yields at low palladium loadings. For an economic process it is necessary to keep the number of additives in the system as low as possible. Preferably only the base would be needed.

It was thus an object of the present invention to provide a process for producing nitro-substituted biphenyls via Suzuki coupling which avoids some of the drawbacks of the prior art processes, especially when these are applied on a large scale. Especially it was the object of the present invention to provide a process for producing substituted biphenyls via Suzuki coupling which requires distinctly lower amounts of palladium, suppresses homocoupling, uses aryl-chlorides, water as solvent and is well-suited for large-scale applications with a simple work-up.

The object is achieved by a process for preparing substituted biphenyls of the formula I 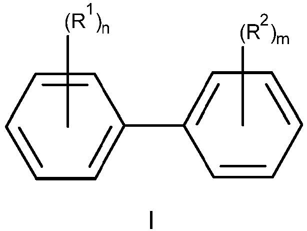 in which the substituents are each defined as follows:
- R¹: is cyano, nitro, F, Cl, C₁-C₄-alkyl, C₁-C₄-haloalkyl, C₃-C₁₀-cycloalkyl which may carry 1, 2, 3 or 4 C₁-C₄-alkyl substituents; C₃-C₁₀-halocycloalkyl, C₁-C₆-alkoxy, C₁-C₆-haloalkoxy, C₁-C₆-alkylcarbonyl, C₁-C₆-haloalkylcarbonyl, C₁-C₆-alkoxycarbonyl, or C₁-C₆-haloalkoxycarbonyl;
- R²: is cyano, nitro, F, Cl, C₁-C₄-alkyl, C₁-C₄-haloalkyl, C₃-C₁₀-cycloalkyl which may carry 1, 2, 3 or 4 C₁-C₄-alkyl substituents; C₃-C₁₀-halocycloalkyl, C₁-C₆-alkoxy, C₁-C₆-haloalkoxy, C₁-C₆-alkylcarbonyl, C₁-C₆-haloalkylcarbonyl, C₁-C₆-alkoxycarbonyl, or C₁-C₆-haloalkoxycarbonyl; and
n is 0, 1, 2 or 3, where, in case that n is 2 or 3, the R¹ radicals may have identical or different definitions; and
m is 0, 1, 2 or 3, where, in case that n is 2 or 3, the R² radicals may have identical or different definitions;
which comprises reacting a compound of the formula II 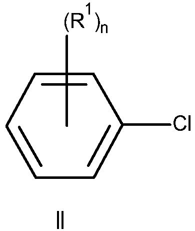 in which R¹ and n is as defined above, in the presence of a base and of a palladium catalyst, where the palladium catalyst is introduced into the reaction in the form of
- a palladium source and a phosphorus ligand of the formula III or a salt thereof 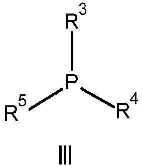 in which
   R³ is C₃-C₁₈-alkyl or C₃-C₁₀-cycloalkyl which is connected to the phosphor atom at a secondary or tertiary carbon atom of the C₃-C₁₈-alkyl or C₃-C₁₀-cycloalkyl substituent
   R⁴ is C₁-C₁₈-alkyl or C₃-C₁₀-cycloalkyl
   and R⁵ is C₁-C₁₈-alkyl or C₃-C₁₀-cycloalkyl or
- a palladium complex containing at least one phosphorus ligand of the formula III as defined above or a salt thereof;
with water as the solvent, wherein the total solvent comprises at least 95wt.-% water based on the total solvent, without the use of an additional surfactant or phase transfer catalyst
with an organoboron compound of the formula IV 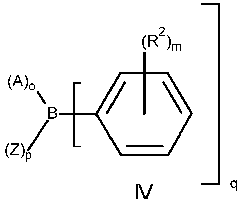 wherein R² and m are as defined above and the compound of formula IV is selected from the group consisting of
(i) boronic acids with o = 0, p = 2; q = 1 and Z = hydroxyl groups, or their trimers;
(ii) boronic acid derivates with o = 0, p = 2; q = 1 and Z = halogen; C₁-C₄-alkyl, C₁-C₆-alkoxy or C₆-C₁₀-aryloxy;
(iii) boronic acids or boronic acid derivatives with o = 0, p = 1; q = 2 and Z = hydroxy, halogen, C₁-C₄-alkyl, C₁-C₆-alkoxy or C₆-C₁₀-aryloxy;
(iv) mixed boronic acids or boronic acid derivatives with o =1, p = 1; q=1, A = C₁-C₄-alkyl and Z = hydroxy, halogen, C₁-C₄-alkyl, C₁-C₆-alkoxy or C₆-C₁₀-aryloxy;
(v) cyclic boronic esters with o = 0, p = 2 and q = 1, wherein the two Z groups form together a bridging group -O-(CH₂)ᵣ-O-, wherein r is 2 or 3, so that the two Z groups, together with the boron atom to which they are attached, form a 5- or 6-membered ring, where the CH₂ groups are optionally substituted by one or two C₁-C₄-alkyl groups;
(vi) boronates with o = 0, p = 3, q = 1 and Z = hydroxyl, halogen, C₁-C₄-alkyl, C₁-C₆-alkoxy or C₆-C₁₀-aryloxy, and accompanied by a cation which compensates the negative charge of the boronate anion;
(vii) triarylboranes with o = 0, p = 0 and q = 3;
(viii) tetraarylborates with o = 0, p = 0 and q = 4, and accompanied by a cation which compensates the negative charge of the borate anion;
where the reaction is carried out at a temperature of from 60 to 150°C.

The organic moieties mentioned in the above definitions of the variables are - like the term halogen - collective terms for individual listings of the individual group members.

The term halogen denotes in each case fluorine, bromine, chlorine or iodine, in particular fluorine, chlorine or bromine.

The term "alkyl" as used herein and in the alkyl moieties of alkoxy, alkylcarbonyl, alkoxycarbonyl and the like refers to saturated straight-chain or branched hydrocarbon radicals having 1 to 2 ("C₁-C₂-alkyl"), 1 to 3 ("C₁-C₃-alkyl"), 1 to 4 ("C₁-C₄-alkyl"), 1 to 6 ("C₁-C₆-alkyl") or 1 to 8 ("C₁-C₈-alkyl") carbon atoms. C₁-C₂-Alkyl is methyl or ethyl. C₁-C₃-Alkyl is additionally propyl and isopropyl. C₁-C₄-Alkyl is additionally n-butyl, 1-methylpropyl (sec-butyl), 2-methylpropyl (isobutyl) or 1,1-dimethylethyl (tert-butyl). C₁-C₆-Alkyl is additionally also, for example, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2.2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl. C₁-C₈-Alkyl is additionally also, for example, heptyl, octyl and the isomers thereof.

The term "haloalkyl" as used herein, which is also expressed as "alkyl which is partially or fully halogenated", refers to straight-chain or branched alkyl groups having 1 to 2 ("C₁-C₂-haloalkyl"), 1 to 3 ("C₁-C₃-haloalkyl"), 1 to 4 ("C₁-C₄-haloalkyl") or 1 to 6 ("C₁-C₆-haloalkyl") carbon atoms (as mentioned above), where some or all of the hydrogen atoms in these groups are replaced by halogen atoms as mentioned above: in particular C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. C₁-C₃-haloalkyl is additionally, for example, 1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 3-chloropropyl and the like. Examples for C₁-C₄-haloalkyl are, apart those mentioned for C₁-C₃-haloalkyl, 4-chlorobutyl and the like.

"Methyl which is substituted by 1, 2 or 3 fluorine atoms" is fluoromethyl, difluoromethyl or trifluoromethyl.
"C₁-C₆-Hydroxyalkyl" is C₁-C₆-alkyl, as defined above, where one hydrogen atom is replaced by a hydroxy group. Examples are hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 1-hydroxybutyl, 2-hydroxybutyl, 3-hydroxybutyl,
4-hydroxybutyl, 1-hydroxypentyl, 2-hydroxypentyl, 3-hydroxypentyl, 4-hydroxypentyl, 5-hydroxypentyl, 1-hydroxyhexyl, 2-hydroxyhexyl, 3-hydroxyhexyl, 4-hydroxyhexyl, 5-hydroxyhexyl, 6-hydroxyhexyl, and the like.

The term "cycloalkyl" as used herein refers to mono- or bicyclic saturated hydrocarbon radicals having 3 to 10 ("C₃-C₁₀-cycloalkyl"), 3 to 8 ("C₃-C₈-cycloalkyl"), in particular 3 to 6 ("C₃-C₆-cycloalkyl") or 3 to 5 ("C₃-C₅-cycloalkyl") or 3 to 4 ("C₃-C₄-cycloalkyl") carbon atoms. Examples of monocyclic radicals having 3 to 4 carbon atoms are cyclopropyl and cyclobutyl. Examples of monocyclic radicals having 3 to 5 carbon atoms are cyclopropyl, cyclobutyl and cyclopentyl. Examples of monocyclic radicals having 3 to 6 carbon atoms are cyclopropyl, cyclobutyl, cyclopentyl and cyclohexyl. Examples of monocyclic radicals having 3 to 8 carbon atoms are cyclopropyl, cyclobutyl, cyclopentyl, cyclohexyl, cycloheptyl and cyclooctyl. Examples of monocyclic radicals having 3 to 10 carbon atoms are cyclopropyl, cyclobutyl, cyclopentyl, cyclohexyl, cycloheptyl, cyclooctyl, cyclononyl and cyclodecyl. The bicyclic radicals can be condensed or bridged rings. Examples of bicyclic condensed radicals having 6 to 10 carbon atoms comprise bicyclo[3.1.0]hexyl, bicyclo[3.2.0]heptyl, bicyclo[3.3.0]octyl (1,2,3,3a,4,5,6,6a-octahydropentalenyl), bicyclo[4.2.0]octyl, bicyclo[4.3.0]nonyl (2,3,3a,4,5,6,7,7a-octahydro-1 H-indene), bicyclo[4.4.0]decyl (decalinyl) and the like. Examples of bridged bicyclic condensed radicals having 7 to 10 carbon atoms comprise bicyclo[2.2.1]heptyl, bicyclo[3.1.1 ]heptyl, bicyclo[2.2.2]octyl, bicyclo[3.2.1]octyl and the like. Preferably, the term cycloalkyl denotes a monocyclic saturated hydrocarbon radical.

The term "halocycloalkyl" as used herein, which is also expressed as "cycloalkyl which is partially or fully halogenated", refers to mono- or bicyclic saturated hydrocarbon groups having 3 to 10 ("C₃-C₁₀-halocycloalkyl") or 3 to 8 ("C₃-C₈-halocycloalkyl") or preferably 3 to 6 ("C₃-C₆-halocycloalkyl") or 3 to 5 ("C₃-C₅-halocycloalkyl") or 3 to 4 ("C₃-C₄-halocycloalkyl") carbon ring members (as mentioned above) in which some or all of the hydrogen atoms are replaced by halogen atoms as mentioned above, in particular fluorine, chlorine and bromine.

"Alkoxy" is an alkyl group attached via an oxygen atom. The term "C₁-C₂-alkoxy" is a C₁-C₂-alkyl group, as defined above, attached via an oxygen atom. The term "C₁-C₃-alkoxy" is a C₁-C₃-alkyl group, as defined above, attached via an oxygen atom.

The term "C₁-C₄-alkoxy" is a C₁-C₄-alkyl group, as defined above, attached via an oxygen atom. The term "C₁-C₆-alkoxy" is a C₁-C₆-alkyl group, as defined above, attached via an oxygen atom. C₁-C₂-Alkoxy is methoxy or ethoxy. C₁-C₃-Alkoxy is additionally, for example, n-propoxy and 1-methylethoxy (isopropoxy). C₁-C₄-Alkoxy is 5 additionally, for example, butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy.

"Haloalkoxy" is a haloalkyl group attached via an oxygen atom. The term "C₁-C₂-haloalkoxy" is a C₁-C₂-haloalkyl group, as defined above, attached via an oxygen atom.

The term "C₁-C₃-haloalkoxy" is a C₁-C₃-haloalkyl group, as defined above, attached via an oxygen atom. The term "C₁-C₄-haloalkoxy" is a C₁-C₄-haloalkyl group, as defined above, attached via an oxygen atom. The term "C₁-C₆-haloalkoxy" is a C₁-C₆-haloalkyl group, as defined above, attached via an oxygen atom. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₄-Haloalkoxy is additionally, for example, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "alkylcarbonyl" is a C₁-C₆-alkyl ("C₁-C₆-alkylcarbonyl"), preferably a C₁-C₄-alkyl ("C₁-C₄-alkylcarbonyl") group, as defined above, attached via a carbonyl [C(=O)] group. Examples are acetyl (methylcarbonyl), propionyl (ethylcarbonyl), propylcarbonyl, isopropylcarbonyl, n-butylcarbonyl and the like.

The term "haloalkylcarbonyl" is a C₁-C₆-haloalkyl ("C₁-C₆-haloalkylcarbonyl"), preferably a C₁-C₄-haloalkyl ("C₁-C₄-haloalkylcarbonyl") group, as defined above, attached via a carbonyl [C(=O)] group. Examples are trifluoromethylcarbonyl, 2,2,2-trifluoroethylcarbonyl and the like.

The term "alkoxycarbonyl" is a C₁-C₆-alkoxy ("C₁-C₆-alkoxycarbonyl"), preferably a C₁-C₄-alkoxy ("C₁-C₄-alkoxycarbonyl") group, as defined above, attached via a carbonyl [C(=O)] group. Examples are methoxycarbonyl, ethoxycarbonyl, propoxycarbonyl, isopropoxycarbonyl, n-butoxycarbonyl and the like.

The term "haloalkoxycarbonyl" is a C₁-C₆-haloalkoxy ("C₁-C₆-haloalkoxycarbonyl"), preferably a C₁-C₄-haloalkoxy ("C₁-C₄-haloalkoxycarbonyl") group, as defined above, attached via a carbonyl [C(=O)] group. Examples are trifluoromethoxycarbonyl, 2,2,2-trifluoroethoxycarbonyl and the like.

If the term "aryl" as used herein and in the aryl moieties of aryloxy is used without prefix (Cₙ-Cₘ), it indicates an aryl group with 6 to 30, in particular 6 to 14, specifically 6 to 10 carbon atoms as ring members. Aryl is a mono-, bi- or polycyclic carbocyclic (i.e. without heteroatoms as ring members) aromatic radical. One example for a monocyclic aromatic radical is phenyl. In bicyclic aryl rings two aromatic rings are condensed, i.e. they share two vicinal C atoms as ring members. One example for a bicyclic aromatic radical is naphthyl. In polycyclic aryl rings, three or more rings are condensed. Examples for polycyclic aryl radicals are phenanthrenyl, anthracenyl, tetracenyl, 1-H-benzo[a]phenalenyl, pyrenyl and the like.

"C₆-C₁₀-Aryl" is phenyl, 1-naphthyl or 2-naphthyl.

"Aryloxy" is aryl, as defined above, bound via an oxygen atom to the remainder of the molecule. "C₆-C₁₀-Aryloxy" is phenoxy, 1-naphthyloxy or 2-naphthyloxy. 5- or 6-membered heteroaryl rings containing 1,2, 3 or 4 heteroatoms selected from the group consisting of N and O as ring members are monocyclic heteroaromatic rings. In the 6-membered heteroaryl rings the heteroatom ring members can only be nitrogen atoms. Examples for 5- or 6-membered heteroaromatic rings containing 1,2,3 or 4 heteroatoms selected from N and O as ring members are 2-furyl, 3-furyl, 1-pyrrolyl, 2-pyrrolyl, 3-pyrrolyl, 1-pyrazolyl, 3-pyrazolyl, 4-pyrazolyl, 5-pyrazolyl, 1-imidazolyl, 2-imidazolyl, 4-imidazolyl, 5-imidazolyl, 2-oxazolyl, 4-oxazolyl, 5-oxazolyl, 3-isoxazolyl, 4-isoxazolyl, 5-isoxazolyl, 1,3,4-triazol-1-yl, 1,3,4-triazol-2-yl, 1,3,4-triazol-3-yl, 1,2,3-triazol-1-yl, 1,2,3-triazol-2-yl, 1,2,3-triazol-4-yl, 1,2,5-oxadiazol-3-yl, 1,2,3-oxadiazol-4-yl, 1,2,3-oxadiazol-5-yl, 1,3,4-oxadiazol-2-yl, tetrazol-1-yl, tetrazol-2-yl, tetrazol-5-yl, 2-pyridinyl, 3-pyridinyl, 4-pyridinyl, 1-oxopyridin-2-yl, 1-oxopyridin-3-yl, 1-oxopyridin-4-yl, 3-pyridazinyl, 4-pyridazinyl, 2-pyrimidinyl, 4-pyrimidinyl, 5-pyrimidinyl, 2-pyrazinyl, 1,3,5-triazin-2-yl, 1,2,4-triazin-3-yl, 1,2,4-triazin-5-yl, 1,2,3,4-tetrazin-1-yl, 1,2,3,4-tetrazin-2-yl, 1,2,3,4-tetrazin-5-yl and the like.

The remarks made below regarding preferred embodiments of the process according to the invention, especially regarding preferred embodiments of the radicals of the different reactants and products (to be more precise preferred embodiments of the variables of the compounds of formulae I, II, III, IV, V and VI, especially with respect to their substituents R¹, R², R³, R⁴, R⁵, Ar and n) and of the reaction conditions of the processes according to the invention, apply either taken alone or, more particularly, in any conceivable combination with one another.

The remarks to preferred embodiments of R¹ apply both to formula I as well as to formula II, V and VI, unless explicitly specified otherwise. The remarks to preferred embodiments of R² and n apply both to formula I as well as to formula IV, V and VI, unless explicitly specified otherwise.

In a particular embodiment, the biphenyl compound I is 4-chloro-2'-nitro-biphenyl, 3,4-dichloro-2'-nitro-biphenyl, 3,4-difluoro-2'-nitro-biphenyl, 3,4,5-trifluoro-2'-nitro-biphenyl, 3-chloro-4,5-difluoro-2'-nitro-biphenyl, 3,4-dichloro-5'-fluoro-2'-nitro-biphenyl or 3,5-dichloro-4-fluoro-2'-nitro-biphenyl. Specifically, the biphenyl compound I is 4-chloro-2'-nitro-biphenyl, 3,4-dichloro-2'-nitro-biphenyl, 3,4,5-trifluoro-2'-nitro-biphenyl or 3,4-dichloro-5'-fluoro-2'-nitro-biphenyl; very specifically 4-chloro-2'-nitro-biphenyl or 3,4,5-trifluoro-2'-nitro-biphenyl.

The organoboron compound IV as defined under (i) in which o = 0, p = 2; q = 1 and Z = OH is a boronic acid of formula IVa. Its trimer is a boroxine and has formula tri-IVa:

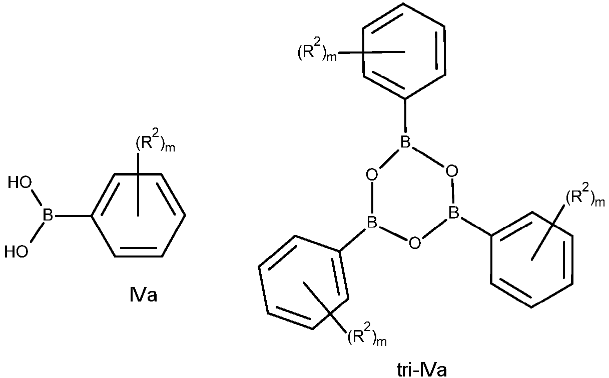

The boronic acid derivates as defined under (ii) with o = 0, p = 2; q = 1 and Z = halogen; C₁-C₄-alkyl, C₁-C₆-alkoxy or C₆-C₁₀-aryloxy are compounds of formula IVb, wherein Z = halogen; C₁-C₄-alkyl, C₁-C₆-alkoxy or C₆-C₁₀-aryloxy:

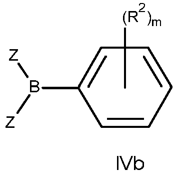

The boronic acids or boronic acid derivatives as defined under (iii) with o = 0, p = 1; q = 2 and Z = hydroxy, halogen, C₁-C₄-alkyl, C₁-C₆-alkoxy or C₆-C₁₀-aryloxy are compounds of formula IVc (boronic acids ) or compounds of formula IVd (boronic acid
derivatives), wherein Z = halogen, C₁-C₄-alkyl, C₁-C₆-alkoxy or C₆-C₁₀aryloxy:

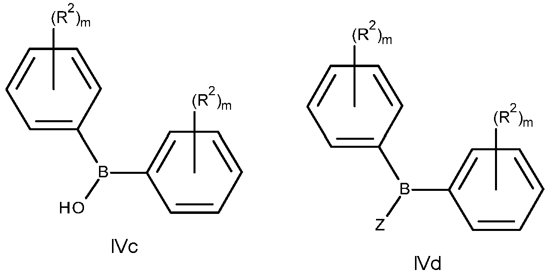

The mixed borinic acids or borinic acid derivatives as defined under (iv) with o =1, p = 1; q=1, A = C₁-C₄-alkyl and Z = hydroxy, halogen, C₁-C₄-alkyl, C₁-C₆-alkoxy or C₆-C₁₀-aryloxy are compounds of formula IVe (mixed borinic acids ) or compounds of formula IVf (mixed boronic acid derivatives), wherein Z = halogen, C₁-C₄-alkyl, C₁-C₆-alkoxy or C₆-C10-aryloxy:

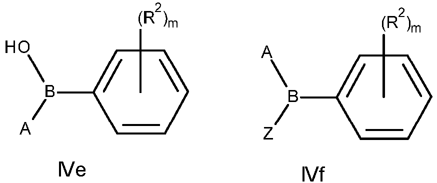

The cyclic boronic esters as defined under (v) with o = 0, p = 2 and q = 1, wherein the two Z groups form together a bridging group -O-(CH₂)ᵣ-O-, wherein r is 2 or 3, so that the two Z groups, together with the boron atom to which they are attached, form a 5- or 6-membered ring, where the CH₂ groups are optionally substituted by one or two C₁-C₄-alkyl groups are compounds of formula IVg: 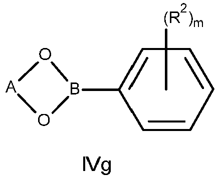 wherein A is -C(R^{A1})(R^{A2})-C(R^{A3})(R^{A4})- or -C(R^{A1})(R^{A2})-C(R^{A3})(R^{A4})-C(R^{A5})(R^{A6})-, where R^{A1}, R^{A2}, R^{A3}, R^{A4}, R^{A5} and R^{A6}, independently of each other, are hydrogen or C₁-C₄-alkyl.

The boronates as defined under (vi) with o = 0, p = 3, q = 1 and Z = hydroxyl, halogen, C₁-C₄-alkyl, C₁-C₆-alkoxy or C₆-C₁₀-aryloxy, and accompanied by a cation which compensates the negative charge of the boronate anion are compounds of formula IVh, wherein each Z is independently hydroxyl, halogen, C₁-C₄-alkyl, C₁-C₆- alkoxy or C₆-C₁₀-aryloxy and (M^{a+})i/a is a cation equivalent:

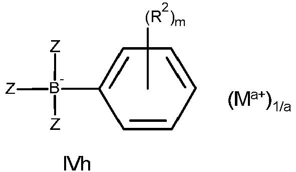

The triarylboranes as defined under (vii) with o = 0, p = 0 and q = 3 are compounds of formula IVi:

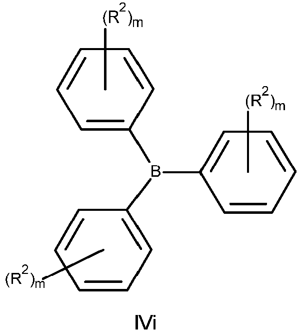

The tetraarylborates as defined under (viii) with o = 0, p = 0 and q = 4, and accompanied by a cation which compensates the negative charge of the borate anion, are compounds of formula IVj, wherein (M^{a+})_{1/a} is a cation equivalent:

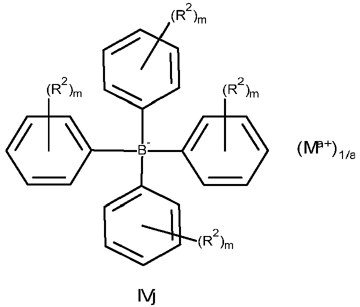

M in compounds IVh and IVj is preferably an alkali or earth alkaline metal cation or an ammonium cation (NR^{a}R^{b}R^{c}R^{d})⁺, wherein R^{a}, R^{b}, R^{c}, and R^{d} independently of each other, are hydrogen, C₁-C₆-alkyl or C₁-C₆-hydroxyalkyl. If M is an alkali metal cation or an ammonium cation, a is 1. If M is an earth alkaline metal cation, a is 2. More preferably, M is an alkali metal cation.

In the above organoboron compounds R² and m have one of the above general or, in particular, one of the above preferred meanings. In a particular embodiment, (R²)ₘ is 4-chloro, 3,4-dichloro, 3,4-difluoro, 3,4,5-trifluoro, 3-chloro-4,5-difluoro or 3,5-dichloro-4-fluoro. Specifically, (R²)ₘ is 4-chloro, 3,4-dichloro or 3,4,5-trifluoro. Very specifically, (R²)ₘ is 4-chloro or 3,4,5-trifluoro.The positions relate to the 1-position of the attachment of the phenyl ring to the boron atom.

A in the mixed boronic acids or boronic acid derivatives as defined under (iv) is in particular methyl. Preferably, the organoboron compound IV is a phenylboronic acid IVa or a diphenylborinic acid IVc 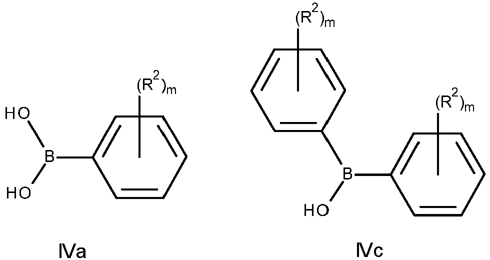 or a mixture of IVa and IVc, in which R² and n have one of the above general or, in particular, one of the above preferred meanings. In particular, the organoboron compound IV is a phenylboronic acid IVa.

In a particular embodiment, (R²)ₘ in IVa and IVc is 4-chloro, 3,4-dichloro, 3,4-difluoro, 3,4,5-trifluoro, 3-chloro-4,5-difluoro or 3,5-dichloro-4-fluoro, more particularly 4-chloro, 3,4-dichloro or 3,4,5-trifluoro, and specifically 4-chloro or 3,4,5-trifluoro. The positions relate to the 1-position of the attachment point of the phenyl ring to the boron atom.

The organoboron compounds as defined under (i) to (viii) and methods for preparing them are known in the art and described, for example, in WO 2015/011032 and the literature cited therein.

The compounds of formulae II and IV are used in a molar ratio of preferably from 5:1 to 1:5, more preferably from 2:1 to 1:2, even more preferably from 1.5:1 to 1:1.5, in particular from 1.1:1 to 1:1.1, specifically from 1.05:1 to 1:1.05, and very specifically of approximately 1:1. "Approximately" is intended to include deviations from ideal stoichiometry caused, for example, by weight errors. Such errors are in general below
10%, mostly below 5%.

The molar ratios of compounds IV as given above relate to the number of phenyl rings contained in the organoboron molecule IV which can react in the Suzuki reaction. Thus, consequently, the molar ratio of compounds II and IVa, IVb, IVe, IVf, IVg or IVh (having one phenyl ring per organoboron molecule which can react in the Suzuki reaction), the compounds IV here counted as such, is preferably from 5:1 to 1:5, more preferably from 2:1 to 1:2, even more preferably from 1.5:1 to 1:1.5, in particular from 1.1:1 to 1:1.1, specifically from 1.05:1 to 1:1.05, and very specifically of approximately1:1; the molar ratio of compounds II and IVc or IVd (having two phenyl rings per organoboron molecule which can react in the Suzuki reaction), the compounds IV here counted as such, is preferably from 10:1 to 1:2.5, more preferably 4:1 to 1:1, even more preferably from 3:1 to 1:0.75, in particular from 2.2:1 to 1:0.55, specifically from 2.1:1 to 1:0.53, and very specifically of approximately 2:1; the molar ratio of compounds II and tri-IVa or IVi (having three phenyl rings per organoboron molecule which can react in the Suzuki reaction), the compounds IV here counted as such, is preferably from 15:1 to 1:1.7, more preferably from 6:1 to 1:0.7, even more preferably from 4.5:1 to 1:0.5, in particular from 3.3:1 to 1:0.37, specifically from 3.15:1 to 1:0.35, and very specifically of approximately 3:1; and the molar ratio of compounds II and IVj (having four phenyl rings per organoboron molecule which can react in the Suzuki reaction), the compound IVj here counted as such, is preferably from 20:1 to 1:1.25, more preferably from 8:1 to 1:0.5, even more preferably from 6:1 to 1:0.38, in particular from 4.4:1 to 1:0.28, specifically from 4.2:1 to 1:0.26, and very specifically of approximately 4:1.

As however the removal of the halogen compound II from the reaction mixture after completion of the reaction is sometimes more difficult than the removal of the organoboron compound IV, it may be advantageous to use the organoboron compound IV in at least equimolar amounts, better in slight excess, so that the halogen compound II is reacted more or less completely. In this case, compounds of formulae II and IV (the latter counted as the number of phenyl rings contained in the organoboron molecule IV which can react in the Suzuki reaction) are used in a molar ratio of preferably from 1:1 to 1:1.5, more preferably from 1:1 to 1:1.1, in particular from 1:1 to 1:1.05 and specifically from 1:1.01 to 1:1.05. However, the inverse stoichiometry is also possible; i.e. compound II can also be used in slight excess; meaning that compounds of formulae II and IV (the latter counted as the number of phenyl rings contained in the organoboron molecule IV which can react in the Suzuki reaction) are used in a molar ratio of preferably from 1:1 to 1.5:1, more preferably from 1:1 to 1.1:1, in particular from 10 1:1 to 1.05:1 and specifically from 1.01:1 to 1.05:1.

Phenyl rings contained in compound IV which can react in the Suzuki reaction are those phenyl rings which are directly bound to the boron atom. Thus, phenyl rings contained in Z, if this is aryloxy, are not counted.

In case of compounds IV, "equimolar amounts" and "excess" amounts are of course related to the number of phenyl rings contained in compounds IV which can react in the Suzuki reaction. In the phosphorus ligands III, R³ is C₃-C₁₈-alkyl or C₃-C₁₀-cycloalkyl which is connected to the phosphor atom at a secondary or tertiary carbon atom of the C₁-C₁₈-alkyl or C₃-C₁₀-cycloalkyl substituent and is in particular selected from 2-propyl, tert-butyl, adamantly, cyclohexyl or cyclopentyl, R⁴ and R⁵ are preferably, independently of each other, C₁-C₁₈-alkyl or C₃-C₁₀-cycloalkyl.

Salts of the phosphorus ligands are acid addition salts, these thus having the formula: 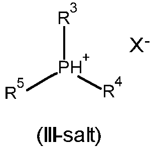 where X- is an anion. Principally any anion derived from a strong acid is suitable, but seeing the desire to avoid certain anions in the waste water, preferred anions are selected from the group consisting of chloride, sulfate, hydrogensulfate, phosphate, hydrogen phosphate, dihydrogenphosphate, perchlorate, tetrafluoroborate, hexafluorophosphate, hydrogenhexafluorozir-conate and hydrogenhexafluorotitanate. Specifically, X- is tetrafluoroborate (BF₄⁻).

The phosphorus ligand III is preferably selected from tBuCy₂P, tBu₂CyP, tBu₃P, Cy₃P, Cy₃P, AdCy₂P, Ad₂nBuP, Ad₂CyP, Ad₃P, Cyp₃P, tBu₂CypP, tBuCyp₂P, tBuiPr₂P, tBu₂iPrP and iPr₃P (Cy = cyclohexyl, Cyp = cylopentyl, Ad = adamantyl, tBu = tert-Butyl, iPr = iso-propyl)

As said above, the palladium catalyst is introduced into the reaction in the form of a palladium source and a phosphorus ligand of the formula III or a salt thereof, or in form of a palladium complex containing at least one phosphorus ligand of the formula III as defined above or a salt thereof.

If the palladium catalyst is introduced into the reaction in the form of a palladium source and a phosphorus ligand of the formula III or a salt thereof, the complex with the ligand (III) is either formed before the Suzuki reaction starts or, in particular, is formed in situ.

The palladium source is preferably a palladium(II) salt, a palladium(0)- or palladium(I) complex compound, a palladium complex with ligands different from the ligand of formula III or its salt or is metallic palladium which is optionally bound to a carrier.

Suitable Pd(ll) salts are for example Pd(II)acetate, Pd(II)benzoate, PdCl₂, Na₂PdCl₄, (NH₄)₂PdCl₄, PdBr₂, PdI₂, Pd(NO₃)₂, PdSO₄,Pd(CF₃COO)₂, Pd(tBuCOO)₂, . Preference is given to Pd(II) acetate and PdCl₂- In particular, Pd(ll) acetate is used.

Suitable palladium(0) or palladium(II) complex compounds are di-µ-iodobis(tri-tertbutylphosphino)dipalladium(I), di-µ-bromobis(tri-tertbutylphosphino)dipalladium(I) bis(dibenzylideneacetone)palladium(0), tris(dibenzylideneacetone)di-palladium(0), bis(tri-tertbutylphosphine)palladium(0), bis(tricyclohexylphosphine)palladium(0), bis(tri-o-tolylphosphine)palladium(0), tetrakis(triphenylphosphine)palladium(0)
Suitable Pd(II) complexes with ligands different from the ligand of formula III or its salt are for example Pd(II) acetylacetonate, bis(2,2,6,6-tetramethyl-3,5-heptanedionateo)palladium(II), palladium(ll)hexafluoracetylacetonate, allyl(cyclopentadienyl)palladium(II), bis(acetonitrile)palladium(ii)(p-toluenesulfonate), allylpalladium(II)chloride dimer, bis(2-methylallyl)palladium(II) chloride dimer, trans-bis(dicyclohexylamine)bis(acetate)palladium(ll), chloromethyl(1,5-cyclooctadiene)palladium(II), dichloro(1,5-cyclooctadiene)palladium(ll), dibro-mo(1,5-cyclooctadiene)palladium(ll), dichloro(norbonadiene)palladium(II), diacetate-bis(triphenylphosphine)palladium(II), dibromo-bis(triphenylphosphine)palladium(ll), dichloro-bis(triphenylphosphine)palladium(II) diacetate(1,10-phenanthroline)palladium(ll), trans-dichlorodiammine palladium(II), cis-dichloro(N, N, N', N'-tetramethylenediamine)palladium(II), bisbenzonitrile Pd(II) chloride or bisacetonitrile Pd(ll) chloride.

A suitable carrier for metallic palladium is charcoal.

The palladium complex containing at least one phosphorus ligand of the formula III as defined above or a salt thereof can be a pre-formed complex of palladium(0) and the ligand III or a salt thereof, or can be a pre-formed palladium(II)-, palladium(I) or palladium(0) complex and the ligand III or a salt thereof.

In case that a Pd(II) salt or a Pd(ll) complex or a Pd(I) complex is used, Pd(ll) is reduced to Pd(0) before the Suzuki reaction starts. The reduction generally takes place in situ.

In one preferred embodiment, the palladium catalyst is introduced into the reaction in form of a palladium(II) salt, specifically Pd(II) acetate or PdCl₂, and the 5 ligand III or a salt thereof.

In another preferred embodiment, the palladium catalyst is introduced into the reaction in form of a pre-formed complex of palladium(0) or (II) and the ligand III or a
salt thereof.

If the palladium catalyst is not introduced into the reaction in form of the preformed complex of palladium and the ligand III, but in form of a Pd source (e.g. a palladium(II) salt, a palladium complex with ligands different from III (or its salt) or a palladium(0) source), and a phosphorus ligand of the formula III or a salt thereof, the Pd source (calculated on the basis of the Pd content) and the ligand of formula III or its salt are used in a molar ratio of preferably from 5:1 to 1:5, more preferably from 2:1 to 1:3, even more preferably from 1.5:1 to 1:2.5, in particular from 1.1:1 to 1:2.5, specifically from 1.05:1 to 1:2.2, very specifically from 1:1 to 1:2.

The Pd catalyst, i.e. the Pd source or the preformed Pd complex containing at least one phosphorus ligand of the formula III as defined above or a salt thereof (in both cases calculated on the basis of the Pd content) can principally be used in an amount of up to 5 mol%, e.g. of from 0.00001 mol% to 5 mol%, relative to 1 mol of compound II or of compound IV (1 mol of compound II or of compound IV corresponding to 100 mol%). If compounds II and IV are not used in equimolar amounts, the above mol% relate to 1 mol of that compound II or IV which is not used in excess. The ligand III or its salt and the other reaction conditions allow however for the use of Pd in significantly lower amounts. Thus, preferably, the Pd catalyst (calculated on the basis of the Pd content) is used in an amount of from 0.00001 mol% to 0.5 mol%, more preferably from 0.00001 mol% to 0.01 mol%, in particular from 0.0001 mol% to 0.001 mol%, and specifically from 0.001 mol% to 0.01 mol%.

If compounds II and IV are not used in equimolar amounts, the above mol% relate to 1 mol of that compound II or IV which is not used in excess. Where the amount of the Pd catalyst is related to the compound IV, the latter is of course counted as the number of phenyl rings contained therein which can react in the Suzuki reaction. In other words, where the amount of the Pd catalyst is related to the compound IV, the amount of the Pd catalyst of course actually relates to 1 mol of phenyl rings contained in compound IV which can react in the Suzuki reaction. Thus, for example, in case of boronic acids IVc, which have two phenyl rings, x mol% Pd, relative to 1 mol of compound IVc, means in this case x mol% Pd relative to 1 mol of phenyl rings contained in IVc, and thus to 0.5 mol of compound IVc taken as such. Phenyl rings contained in compound IV which can react in the Suzuki reaction are those phenyl rings which are directly bound to the boron atom. In case of compounds IV, "equimolar amounts" and "excess" amounts are of course related to the number of phenyl rings contained in compounds IV which can react in the Suzuki reaction.

The reaction is carried out in water as the main solvent, preferably the content of water in the total solvent is 95%, more preferably 97% and most preferred 98% by weight. In a preferred embodiment of the invention no other solvent miscible with water is added.

Organic solvents which are only partially or not miscible with water can be present in small amounts, i.e. less than 5%, more preferred less than 3% and most preferred less than 2 % of weight of total solvent. These organic solvents are introduced into the reaction mixture usually together with the catalyst and are selected from alkanes, aromatics or esters. These solvents can be linear alkanes as pentane, hexane, heptane, cyclic alkanes as cyclohexane or cyclohexane, or aromatics like benzene, toluene or xylenes or esters as ethyl acetate or butyl acetate.

In the preferred embodiment of the invention, no surfactants or phase transfer catalysts are added to the reaction.

The Suzuki reaction is carried out in the presence of a base. Suitable are both inorganic and organic bases. Suitable inorganic bases are for example from alkali metal carbonates, e.g. Li₂CO₃, Na₂CO₃, K₂CO₃ or Cs₂CO₃, earth alkaline metal carbonates, e.g. MgCO₃ or CaCO₃, alkali metal phosphates, e.g. Li₃PO₄, Na₃PO₄, K₃PO₄ or Cs₃PO₄, earth alkaline metal phosphates, e.g. Mg₃(PO₄)₂ or Ca₃(PO₄)₂, alkali metal hydrogen phosphates, e.g. Li₂HPO₄, Na₂HPO₄, K₂HPO₄ or Cs₂HPO₄, earth alkaline metal hydrogenphosphates, e.g. MgHPO₄ or CaHPO₄, alkali metal hydroxides, LiOH, NaOH or KOH, and earth alkaline metal hydroxides, e.g. Mg(OH)₂ or Ca(OH)₂.

Examples for suitable organic bases are open-chained amines, e.g. trimethylamine, triethylamine, tripropylamine, ethyldiisopropylamine and the like, or basic N-heterocycles, such as morpholine, pyridine, lutidine, DABCO, DBU or DBN. Preference is however given to inorganic bases, such as to the above alkali metal carbonates, earth alkaline metal carbonates, alkali metal phosphates, earth alkaline metal phosphates, alkali metal hydrogen phosphates, earth alkaline metal hydrogenphosphates, alkali metal hydroxides and earth alkaline metal hydroxides.

More preference is given to alkali metal carbonates, alkali metal phosphates and alkali metal hydroxides. Even more preferred are alkali metal carbonates and hydroxides, such as the above-mentioned Na₂CO₃, K₂CO₃, NaOH or KOH.

In view of corrosive properties of carbonates and hydroxides under certain conditions, it may however be more advantageous to use one of the above-listed phosphates. Thus, in an alternative even more preferred embodiment, alkali metal phosphates, such as the above-mentioned Na₃PO₄, K₃PO₄ are used.

The base is preferably used in an amount 0.5 to 5 mol per mol of compound II or of compound IV, more preferably from 1 to 4 mol per mol of compound II or of compound IV, in particular from 1 to 3 mol per mol of compound II or of compound IV, specifically from 1 to 2.2 mol per mol of compound II or of compound IV, and very specifically from 1 to 2 mol per mol of compound II or of compound IV. If compounds II and IV are not used in equimolar amounts, the above relation is to 1 mol of that compound II or IV which is not used in excess. Where the amount of the base is related to the compound IV, the latter is of course counted as the number of phenyl rings contained therein which can react in the Suzuki reaction. In other words, where the amount of the base is related to the compound IV, the amount of the base of course actually relates to 1 mol of phenyl rings contained in compound IV which can react in the Suzuki reaction. Thus, for example, in case of boronic acids IVc, which have two phenyl rings, x mol% of base, relative to 1 mol of compound IVc, means in this case x mol% of base relative to 1 mol of phenyl rings contained in IVc, and thus to 0.5 mol of compound IVc taken as such.

As said above, phenyl rings contained in compound IV which can react in the Suzuki reaction are those phenyl rings which are directly bound to the boron atom. In case of compounds IV, "equimolar amounts" and "excess" amounts are of course related to the number of phenyl rings contained in compounds IV which can react in the Suzuki reaction. The reaction is preferably carried out at a temperature of from 60 to 150°C; more preferably from 90 to 110°C.

The reaction pressure is principally not critical. As however elevated temperatures are used and in case that the solvents used have a boiling point beneath the desired temperature, the reaction is in this case generally carried out in a closed vessel. This results in an inherent pressure, which is generally in the range of from 1.1 to 10 bar, in particular from 1.5 to 5 bar, specifically from 2 to 4 bar.

The reaction can be carried out by standard proceedings for Suzuki reactions, e.g. by mixing all reagents, inclusive catalyst or catalyst precursor and ligand, base and the solvent mixture, and reacting them at the desired temperature. Alternatively, the reagents can be added gradually, especially in the case of a continuous or semicontinuous process.

The reaction is preferably carried out in an inert atmosphere in order to avoid the presence of oxygen, e.g. under an argon or nitrogen atmosphere.

The reaction is preferably carried out in a pressure vessel, e.g. an autoclave. After completion of the reaction, the reaction mixture is worked up and the compound of the formula I is isolated in a customary manner.

For example, the solvents are removed, for example under reduced pressure. Preferably, however, the work-up is carried out by adding a non-polar extraction solvent to the reaction mixture, to extract the biaryl-product from the aqueous phase and leaving the salt by-products in the aqueous phase.

Non-polar organic solvents in terms of the present invention are those which have a miscibility with water of below 20 g /100 g of water at 20°C. Examples are aliphatic hydrocarbons, such as alkanes, e.g. pentane, hexane, heptane, octane, mixtures thereof and technical mixtures, such as petrol ether; cycloaliphatic hydrocarbons, such as cycloalkanes, e.g. cyclohexane, cycloheptane, or cyclooctane; chlorinated aliphatic hydrocarbons, such as halogenalkanes, e.g. dichloromethane, trichloromethane, tetrachloromethane, dichloroethane or tetrachloroethane, aromatic hydrocarbons, such as benzene, toluene, the xylenes, ethylbenzene, cumene (isopropylbenzene), chlorobenzene, o-dichlorobenzene or nitrobenzene, open-chained, ethers, such as diethylether, dipropylether, methyl-tert-butylether or methylisobutylether, higher alkanols, such as n-butanol or isobutanol or esters as ethylacetate or butylacetate.

The product I is after the extraction in the organic phase mainly formed by the non-polar organic solvent. Moreover, the organic phase also contains the Pd catalyst. To enhance the yield, the aqueous phase can be extracted once or more times with an organic solvent, such as the above listed non-polar organic solvents. If desired, the product I can then be separated from the catalyst and optionally from other undesired components, such as unreacted starting compounds II and/or IV, via customary means. For example, the compound I is crystallized from the organic phase. Alternatively, the solvent is removed from the organic phase, e.g. by distillation, e.g. under vacuum, optionally after drying the organic phase, and the solid matter is taken up in another solvent in which the compound I crystallizes better.

In yet another alternative, the solid matter is submitted to a chromatographic separation.

Further purification of the product I can be effected if desired; for example by extraction, crystallization, distillation or by chromatography.

If desired, the compound I can be left in the aqueous phase without any further work-up and directly be converted into products of value, such as carboxamides of formula V or the isolated and purified compound I can be used to synthesize products of value such as carboxamides of formula V. 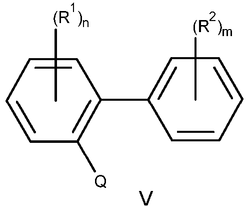 where R¹, R² and n have one of the above general or, in particular, one of the above preferred meanings, and Q is Q¹, Q² or Q³ 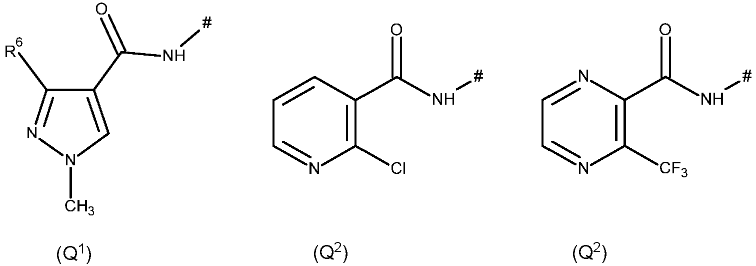 with R⁶ being methyl, optionally substituted by 1,2 or 3 fluorine atoms, and # being the attachment point to the remainder of the molecule;
which process comprises
(a) preparing a compound of the formula I as defined above with a process as defined above where one R¹ is a nitro group;
(b) reducing the nitro group of the compound of formula I obtained in step (a) to an amino group to obtain a compound of the formula VI 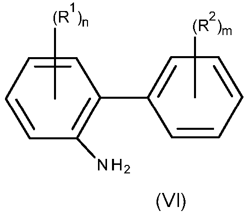
and reacting the amino compound of the formula VI with a compound Q¹¹, Q²¹ or Q³¹ 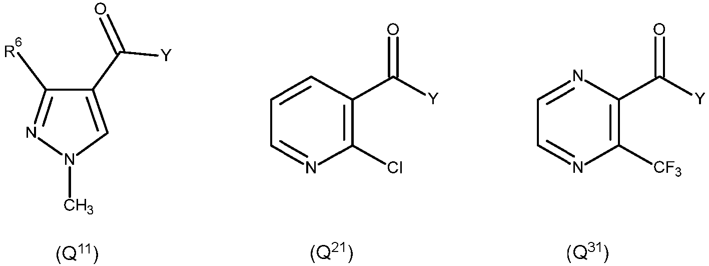 where R⁶ is as defined above and Y is a leaving group.

Reduction in step (b) may be carried out with hydrogen in the presence of a hydrogenation catalyst, such as Pt on charcoal, or with other reduction agents, such as SnCl₂/HCl, Fe/HCl or Fe/NH₄Cl.

Reduction can be carried out according to known methods of converting aromatic nitro compounds into the corresponding aromatic amino compounds, such as described, for example, in R. J. Rahaim, R. E. Maleczka (Jr.), Org. Lett., 2005, 7, 5087-10 5090, G. S. Vanier, Synlett, 2007, 131-135, S. Chandrasekhar, S. Y. Prakash, C. L. Rao, J. Org. Chem., 2006, 71,2196-2199, H. Berthold, T. Schotten, H. Honig, Synthesis, 2002, 1607-1610, and C. Yu, B. Liu, L. Hu, J. Org. Chem., 2001, 66, 919-924.

To obtain compounds V, the amino compound VI is subjected in step (c) to an N-acylation with an acyl precursor Q¹¹, Q²¹ or Q³¹.

Suitable leaving groups Y are -OH, a halide, especially chloride or bromide, -OR^{A}, or -O-C(O)-R^{B}.

If compounds Q¹¹, Q²¹ or Q³¹ are acids, i.e. Y = OH, the reaction can be performed in the presence of a coupling reagent. Suitable coupling reagents (activators) are well known in the art.

If Y = halide, the reaction is expediently performed in the presence of a base. Suitable bases are those listed above in context with the Suzuki coupling.

If Y = OR^{A}, the compounds Q¹¹, Q²¹ or Q³¹ are esters. Suitable esters derive preferably from C₁-C₄-alkanols R^{A}OH in which R^{A} is C₁-C₄-alkyl, or from C₂-C₆-polyols such as glycol, glycerol, trimethylolpropane, erythritol, pentaerythritol and sorbitol.

Alternatively, the ester is a so-called active ester, which is obtained in a formal sense
by the reaction of the acid Q¹¹, Q²¹ or Q³¹ (Y = OH) with an active ester-forming alcohol, such as p-nitrophenol, N-hydroxybenzotriazole (HOBt), N-hydroxysuccinimide or OPfp (pentafluorophenol).

If compounds Q¹¹, Q²¹ or Q³¹ are anhydrides, i.e. Y = O-C(O)-R^{B}, these are either a symmetric anhydride or an asymmetric anhydride in which -O-OC-R^{B} is a group which can be displaced easily by the 2-aminobiphenyl (VI) used in the reaction.

Suitable acid derivatives with which the carboxylic acid Q¹¹, Q²¹ or Q³¹ with Y = OH can form suitable mixed anhydrides are, for example, the esters of chloroformic acid, for example isopropyl chloroformate and isobutyl chloroformate, or of chloroacetic acid. The acylation can be carried out under known conditions.

The method of the invention yields compounds I in high yields, although an aromatic

chloride is used instead of the generally more reactive aromatic bromides or iodides, as used for example in WO 2015/011032. Moreover, the method requires distinctly lower amounts of Pd than most prior art processes. The Suzuki reaction proceeds very selectively, effectively suppressing homocoupling reactions. The process is very well suited for large scale production, and the workup is very simple. By the choice of ligands according formula III, no water miscible organic solvent is required in the reaction, which makes the work up and isolation of the product even more simple than with the system described in WO 2018/149813. Moreover, as the required amounts of Pd are so low, the catalyst does not need to be recycled, which is a very time-consuming and costly procedure, but can be disposed of after the reaction.

The invention is further illustrated by the following examples.

### Examples

• Investigation on the surfactant effects

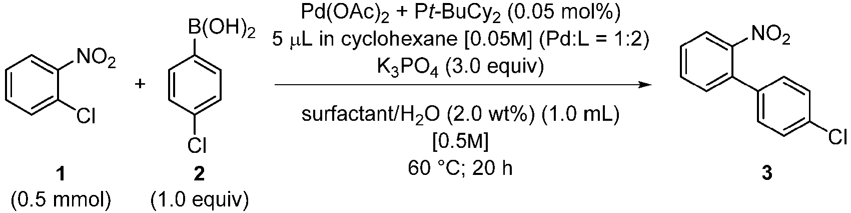

| entry | surfactant | conversion [%]^{[b]} | yield [%]^{[b]} |
|---|---|---|---|
| 1*) | SPGS-550-M | >99 | 93 |
| 2*) | TPGS-750-M | >99 | 95 |
| 3*) | Brij 30 | >99 | 87 |
| 4*) | Brij S100 | >99 | 93 |
| 5*) | IGEPAL CA720 | >99 | 90 |
| 6*) | Sorbitan monopalmitate | >99 | 70 |
| 7*) | Sodium Dodecyl Sulfate | >99 | 89 |
| 8*) | Benzethonium chloride | >99 | 88 |
| 9 | no surfactant | >99 | 97 (93)^{c} |

[a] Unless otherwise noted, all reactions were performed in a glovebox with 0.50 mmol of the aryl halide **1,** 1.0 equiv of 4-chlorophenylboronic acid **2,** K₃PO₄ (3.0 equiv) and 10 µL of Pd(OAc)₂ + P*t*-BuCy₂ solution (0.05M in cyclohexane) (0.10 mol%) in 1.0 mL of the respective surfactant in H₂O (2.0 wt%) at 50 °C for 20 h. [b] Determined by GC analysis after calibration, using mesitylene as internal standard. [c] Isolated yield.
*) not according to the invention
• Screening of tri-alkylphosphines at [Pd] ppm level under optimized conditions

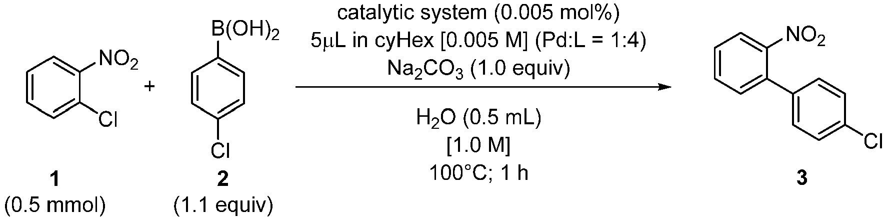

| entry | catalytic system | conversion [%]^{[b]} | yield [%]^{[b]} |
|---|---|---|---|
| 1 | Pd(OAc)₂ + PCy₃ | 61 | 44^{[c]} |
| 2 | Pd(OAc)₂ + P*t*Bu₃ | 60 | 45 |
| 3*) | Pd(OAc)₂ + P*n*Bu₃ | 28 | 5 |
| 4 | Pd(OAc)₂ + PAd₃ | 78 | 62 |
| 5*) | Pd(OAc)₂ + PMe₃ | 23 | 8^{[d]} |
| 6*) | Pd(OAc)₂ + P*n*Oct₃ | 36 | 10 |
| 7 | Pd(OAc)₂ + PAd₂*n*Bu | >99 | 88 |
| 8*) | Pd(OAc)₂ + PBn₃ | 25 | 3^{[c]} |
| 9 | Pd(OAc)₂ + PCp₃ | 48 | 31 |

[a] Unless otherwise noted, all reactions were performed in a glovebox with 0.5 mmol of the aryl halide **1,** 0.55 mmol of the boronic acid **2,** 0.5 mmol of Na₂CO₃ (1.0 equiv) and 5µL of the respective catalytic system (Pd:L = 1:4) in cyclohexane (0.005 mol%), in 0.5 mL of H₂O at 100°C for 1 h. [b] Determined by GC analysis using mesitylene as internal standard. [c] In the GC chromatogram is present a peak at 23.77 min. [d] The stock solution presented solid precipitate.
*) not according to the invention

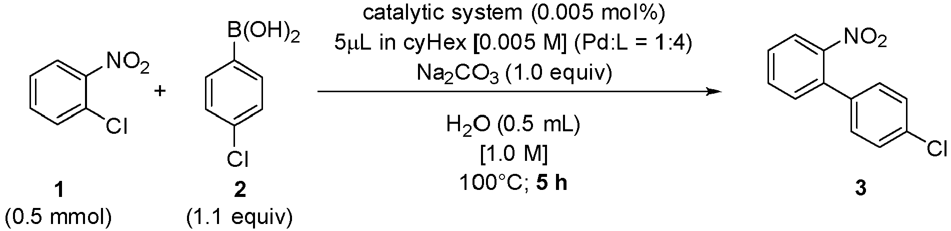

| entry | catalytic system | conversion [%]^{[b]} | yield [%]^{[b]} |
|---|---|---|---|
| 1 | Pd(OAc)₂ + PCy₃ | 96 | 82 (75)^{[c]} |
| 2 | Pd(OAc)₂ + P*t*Bu₃ | >99 | 80 (85)^{[c,d]} |
| 3*) | Pd(OAc)₂ + P*n*Bu₃ | 35 | 13 |
| 4*) | Pd(OAc)₂ + PnOCt₃ | 39 | 12 |
| 5*) | Pd(OAc)₂ + PMe₃ | 23 | 2^{[e]} |

[a] Unless otherwise noted, all reactions were performed in a glovebox with 0.5 mmol of the aryl halide **1,** 0.55 mmol of the boronic acid **2,** 0.5 mmol of Na₂CO₃ (1.0 equiv) and 5µL of the respective catalytic system (Pd:L = 1:4) in cyclohexane (0.005 mol%), in 0.5 mL of H₂O at 100°C for 5 h. [b] Determined by GC analysis using mesitylene as internal standard. [c] Isolated yield. [d] The isolated product contains also another unknown species. [e] The stock solution presented a solid precipitate.
*) not according to the invention

### Experiments (entry 1-5) which are not according to the invention (for comparison)

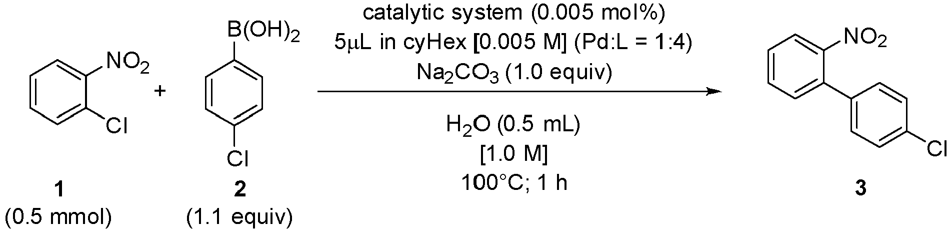

| entry | catalytic system | conversion [%]^{[b]} | yield [%]^{[b]} |
|---|---|---|---|
| 1 | Pd(OAc)₂ + PPh₃ | 25 | 2 |
| 2 | Pd(OAc)₂ + PPhCy₂ | 53 | 33 |
| 3^{c} | Pd(OAc)₂ + PPhCy₂ | 53 | 28 |
| 4 | Pd(OAc)₂ + PPh₂Cy | 30 | 4 |
| 5 | Pd(OAc)₂ + SPhos | 36 | 16 |

[a] Unless otherwise noted, all reactions were performed in a glovebox with 0.5 mmol of the aryl halide **1,** 0.55 mmol of the boronic acid **2,** 0.5 mmol of Na₂CO₃ (1.0 equiv) and 5mL of the respective catalytic system (Pd:L = 1:4) in cyclohexane (0.005 mol%), in 0.5 µL of H₂O at 100°C for 5 h. [b] Determined by GC analysis using mesitylene as internal standard. [c] The reaction was run for 5 h.
• Effect of different solvent using Na₂CO₃ as base with Pd(OAc)₂ + P*t*BuCy₂ (0.005 mol%):

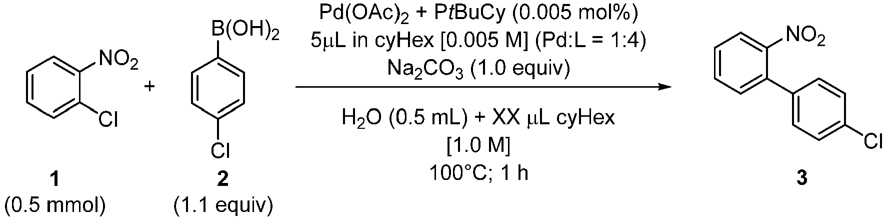

| entry | cyHex addition (µL) | conversion [%]^{[b]} | yield [%]^{[b]} |
|---|---|---|---|
| 1 | none | >99 | 91 |
| 2 | 10 | >99 | 91 |
| 3 | 100 | 87 | 62 |

[a] Unless otherwise noted, all reactions were performed in a glovebox with 0.5 mmol of the aryl halide **1,** 0.55 mmol of the boronic acid **2,** 0.5 mmol of NaOH (1.0 equiv) and 5µL of Pd(OAc)₂ + P*t*BuCy₂ solution (Pd:L = 1:4) in cyclohexane (0.005 mol%), in 0.5 mL of H₂O at 100°C for 1 h. [b] Determined by GC analysis using mesitylene as internal standard.

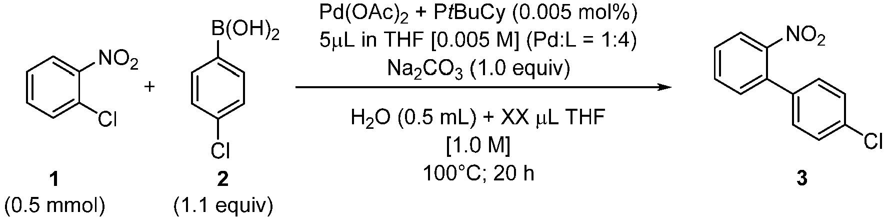

| entry | THF addition (µL) | conversion [%]^{[b]} | yield [%]^{[b]} |
|---|---|---|---|
| 1 | none | >99 | 86 |
| 2 | 10 | >99 | 90 |
| 3 | 100 | >99 | 93 (95)^{c} |

[a] Unless otherwise noted, all reactions were performed in a glovebox with 0.5 mmol of the aryl halide **1,** 0.55 mmol of the boronic acid **2,** 0.5 mmol of NaOH (1.0 equiv) and 5µL of Pd(OAc)₂ + P*t*BuCy₂ solution (Pd:L = 1:4) in tetrahydrofurane (0.005 mol%), in 0.5 mL of H₂O at 100°C for 20 h. [b] Determined by GC analysis using mesitylene as internal standard. [c] Isolated yield.

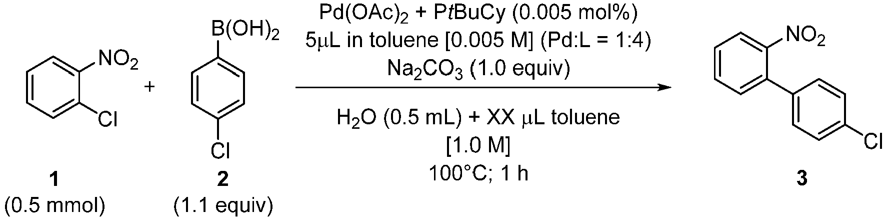

| entry | toluene addition (µL) | conversion [%]^{[b]} | yield [%]^{[b]} |
|---|---|---|---|
| 1 | none | >99 | 89 |
| 2 | 10 | >99 | 90 |
| 3 | 100 | >99 | 93 |

[a] Unless otherwise noted, all reactions were performed in a glovebox with 0.5 mmol of the aryl halide **1,** 0.55 mmol of the boronic acid **2,** 0.5 mmol of NaOH (1.0 equiv) and 5µL of Pd(OAc)₂ + P*t*BuCy₂ solution (Pd:L = 1:4) in toluene (0.005 mol%), in 0.5 mL of H₂O at 100°C for 1 h. [b] Determined by GC analysis using mesitylene as internal standard.

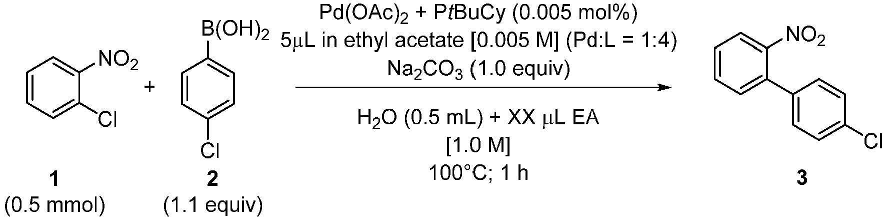

| entry | EA addition (µL) | conversion [%]^{[b]} | yield [%]^{[b]} |
|---|---|---|---|
| 1 | none | >99 | 97 (91)^{[c]} |
| 2 | 10 | >99 | 86 |
| 3 | 100 | >99 | 97 |

[a] Unless otherwise noted, all reactions were performed in a glovebox with 0.5 mmol of the aryl halide **1,** 0.55 mmol of the boronic acid **2,** 0.5 mmol of NaOH (1.0 equiv) and 5µL of Pd(OAc)₂ + P*t*BuCy₂ solution (Pd:L = 1:4) in ethyl acetate (0.005 mol%), in 0.5 mL of H₂O at 100°C for 1 h. [b] Determined by GC analysis using mesitylene as internal standard. [c] Isolated yield.
• Temperature screening under optimized conditions:

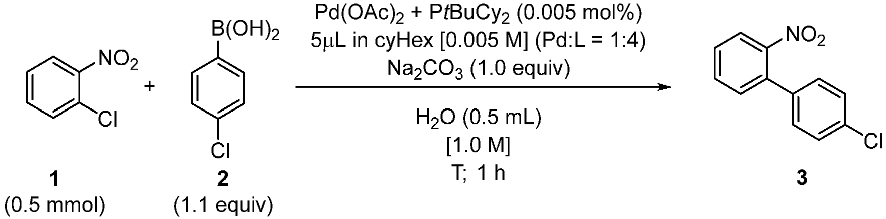

| entry | temperature (°C) | conversion [%]^{[b]} | yield [%]^{[b]} |
|---|---|---|---|
| 1 | 80 | >99 | 81 |
| 2 | 90 | >99 | 84 |
| 3 | 120 | >99 | 78 |
| 4 | 140 | >99 | 80^{[c]} |

[a] Unless otherwise noted, all reactions were performed in a glovebox with 0.5 mmol of the aryl halide **1,** 0.55 mmol of the boronic acid **2,** 0.5 mmol of Na₂CO₃ (1.0 equiv) and 5µL of Pd(OAc)₂ + P*t*BuCy₂ (Pd:L = 1:4) in cyclohexane (0.005 mol%), in 0.5 mL of H₂O at the given temperature for 1 h. [b] Determined by GC analysis using mesitylene as internal standard.
• Investigation of SUZUKI-MIYAURA cross-coupling at lower [Pd ] ppm level:

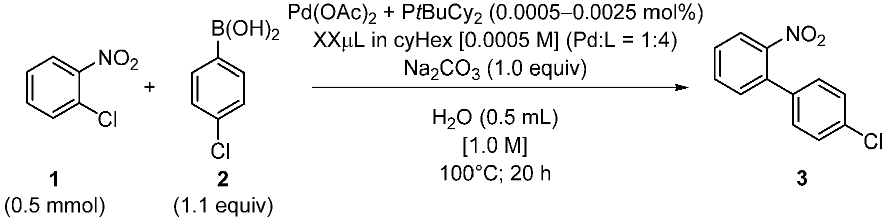

| entry | µL of catalyst added | conversion [%]^{[b]} | yield [%]^{[b]} |
|---|---|---|---|
| 1 | 5 (0.0005 mol%) | 65 | 35 |
| 2 | 10 (0.001 mol%) | 75 | 51 |
| 3 | 25 (0.0025 mol%) | 81 | 67 |

[a] Unless otherwise noted, all reactions were performed in a glovebox with 0.5 mmol of the aryl halide **1,** 0.55 mmol of the boronic acid **2,** 0.5 mmol of Na₂CO₃ (1.0 equiv) and the respective µL of Pd(OAc)₂ + P*t*BuCy₂ in cyclohexane (Pd:L = 1:4), in 0.5 mL of H₂O at 100°C for 20 h. [b] Determined by GC analysis using mesitylene as internal standard.
• Kinetic experiment on SUZUKI-MIYAURA cross coupling at 50 ppm catalyst loading: 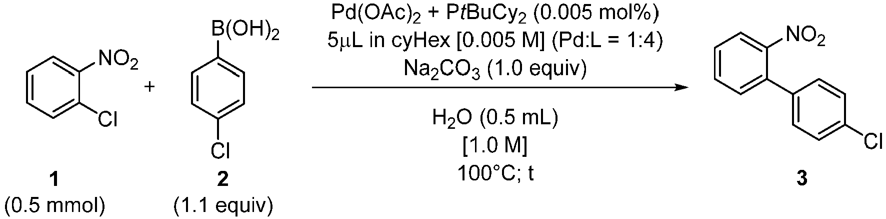 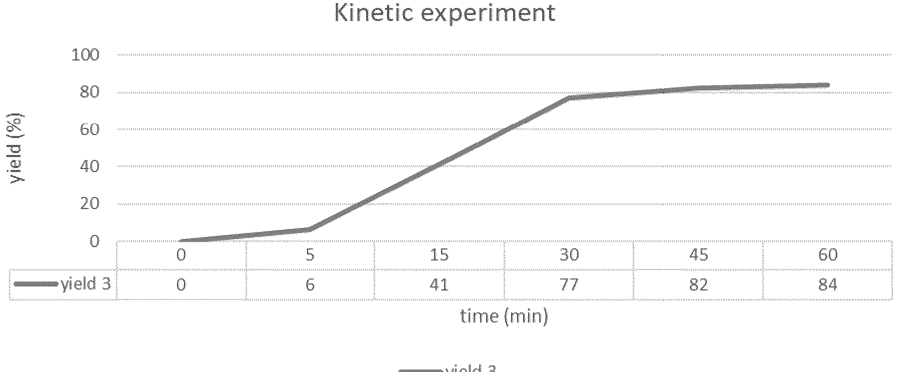 NOTE: 5 different reactions were set at the same t₀. After dedicated time, the respective tube was opened and analyzed by GC using mesitylene as internal standard. After 60 min isolated 103.6mg of yellow solid (89%).
• Investigation with 1.0 equiv of base:

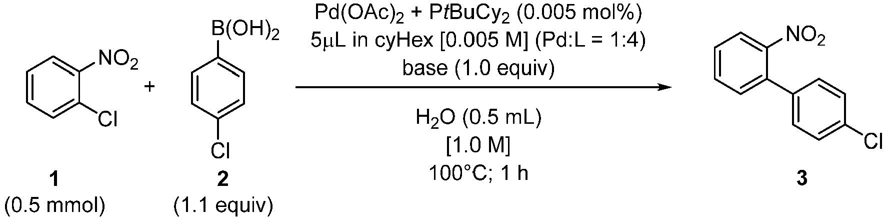

| entry | base | conversion [%]^{[b]} | yield [%]^{[b]} |
|---|---|---|---|
| 1 | K₃PO₄ | 89 | 68 |
| 2 | K₂CO₃ | >99 | 83 |
| 3 | NaOH | 97 | 81 |
| 4 | KOH | 82 | 64 |

[a] Unless otherwise noted, all reactions were performed in a glovebox with 0.5 mmol of the aryl halide **1,** 0.55 mmol of the boronic acid **2,** 0.5 mmol of respective base (1.0 equiv) and 5µL of Pd(OAc)₂ + P*t*BuCy₂ (Pd:L = 1:4) in cyclohexane (0.005 mol%), in 0.5 mL of H₂O at 100°C for 1 h. [b] Determined by GC analysis using mesitylene as internal standard.
• Test experiment removing cyclohexane from the reaction mixture: 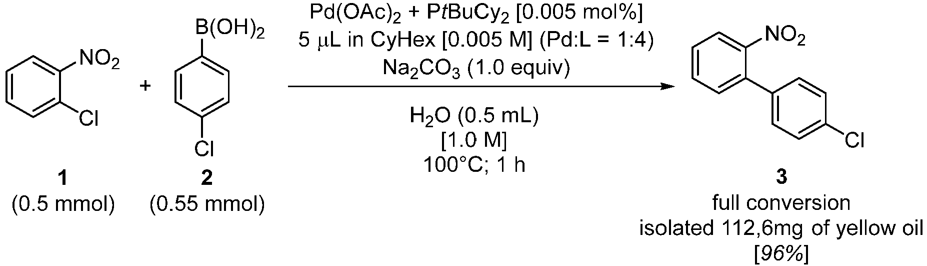 NOTE: the pressure tube was transferred outside the glovebox closed with a rubber septum on top. From the Schlenk line it was applied full vacuum for ca. 10 sec. Presumably, the cyclohexane has been removed.
• Test reaction using a different borane reagent: 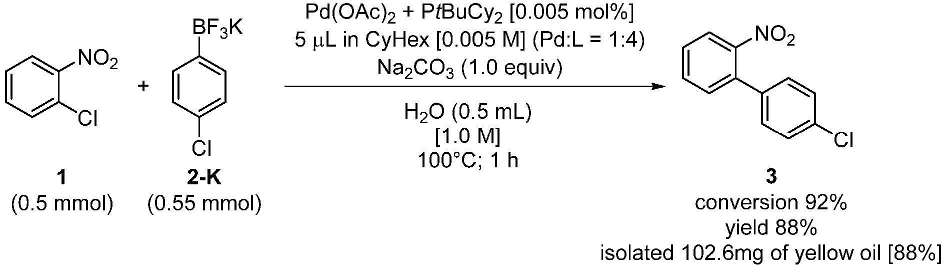
• GCL analysis
GLC analyses were performed using an AGILENT 6890N equipped with a DB-5 capillary column (30 m × 0.32 mm, 0.25 µm, He carrier gas, 250°C injection temperature, detector temperature 250°C; temperature program: start temperature 60°C for 1 min, heating rate 5°C·min⁻¹ until 120°CIIIIII, then 15°C·min⁻¹ until 270°C, end temperature 270°C for 2 min).
tᵣ= 21.01 min
• Test reaction using different Pd sources:
➢ [Pd(allyl)Cl]₂ as Pd(0) catalyst

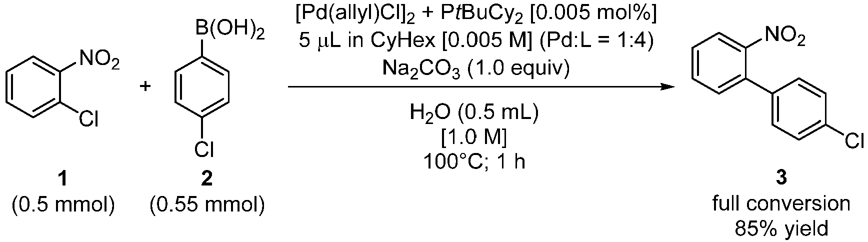

• General procedure for cross-coupling reaction
• General Procedure for the Preparation of the Catalyst Stock solutions

In a glovebox, a 10.0 mL vial was charged with 6.0 mg of Pd(OAc)₂ (0.25 mmol) and 25.0 mg of P*t*-BuCy₂ (0.10 mmol, Pd:L = 1:4) and diluted in 5.0 mL of the respective solvent, obtaining a 0.005M solution of the catalytic system. The mixture was left stirring in the glovebox and the desired amount (5 µL, 0.002 µmol) was taken with a micropipette and directly injected in the reaction vessel.
- General Procedure for Suzuki-Miyaura Cross-Coupling of Aryl Chloride with Aryl Boronic

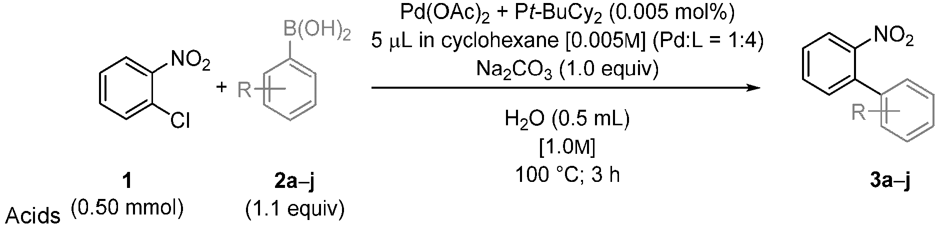

In a glovebox, a 2.0 mL pressure tube was equipped with a magnetic stir bar, 0.55 mmol of the corresponding aryl boronic acid **(2a-2j)** was added with 0.50 mmol of Na₂CO₃ (53.0 mg, 1.0 equiv), 5 µL of Pd(OAc)₂ and P*t*BuCy₂ solution (0.005M in cyclohexane) (0.002 µmol, 0.005 mol%, Pd:L = 1:4) and 0.50 mmol of 1-chloro-2-nitrobenzene (79.0 mg, 1.0 equiv). The tube was sealed and transferred outside the glovebox, where 0.50 mL of H₂O were added under a flow of Ar. The tube was finally placed in a preheated 100 °C oil bath for 3 h. After this time, the mixture was diluted with 5.0 mL of brine and extracted with EtOAc (3 x 2.0 mL). The combined organic phases were dried over MgSO₄ and filtered. After concentration under reduce pressure, the crude product was purified by flash column chromatography on silica gel using petrol ether:ethyl acetate = 95:5 as eluent to get spectroscopically pure product.

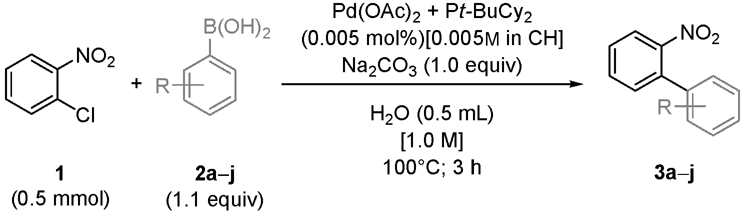

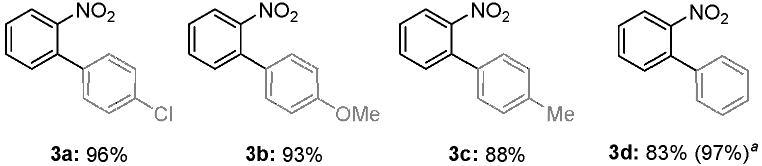

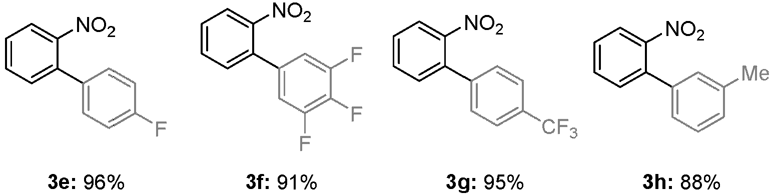

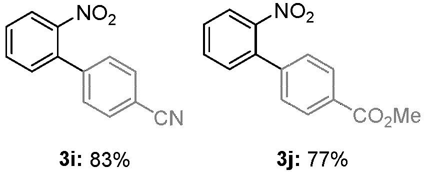

Unless otherwise noted, all reactions were performed in a glovebox with 0.5 mmol of the aryl chloride **1,** 1.1 equiv of the respective boronic acid **2a-j,** Na₂CO₃ (1.0 equiv) and 5mL of Pd(OAc)₂ + P*t*-BuCy₂ solution in cyclohexane (0.005 mol%) [Pd:L = 1:4], in 0.5mL of H₂O at 100°C for 3 h. [a] The reaction was set with 10 µL of Pd(OAc)₂ + P*t*-BuCy₂ solution in cyclohexane (0.010 mol%) [Pd:L = 1:4].
- General Procedure for Suzuki-Miyaura Cross-Coupling of Aryl Halides

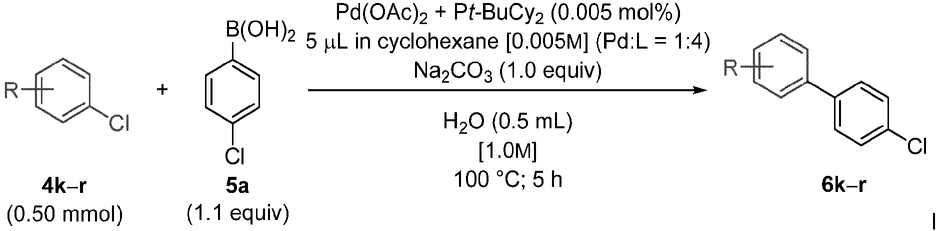

n a glovebox, a 2.0 mL pressure tube was equipped with a magnetic stir bar, 0.55 mmol of 4-chlorophenylboronic acid **(5a)** was added with 0.50 mmol of Na₂CO₃ (53.0 mg, 1.0 equiv), 5 µL of Pd(OAc)₂ and P*t*-BuCy₂ solution (0.005M in cyclohexane) (0.002 µmol, 0.005 mol%, Pd:L = 1:4) and 0.50 mmol of the corresponding aryl chloride **(4k-r).** The tube was sealed and transferred outside the glovebox, where 0.50 mL of H₂O were added under a flow of Ar. The tube was finally placed in a preheated 100 °C oil bath for 5 h. After this time, the mixture was diluted with 5.0 mL of brine and extracted with EtOAc (3 x 2.0 mL). The combined organic phases were dried over MgSO₄ and filtered. After concentration under reduce pressure, the crude product was purified by flash column chromatography on silica gel using petrol ether:ethyl acetate = 95:5 as eluent to get spectroscopically pure product.

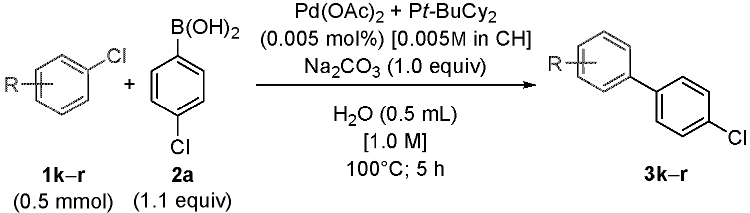

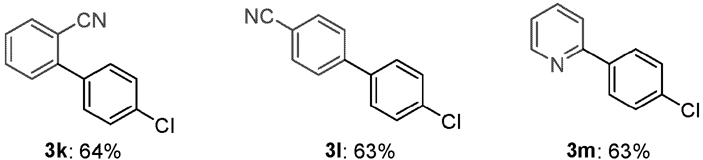

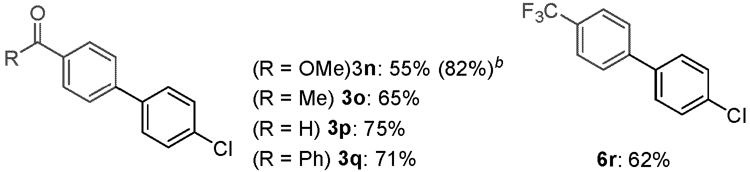

Unless otherwise noted, all reactions were performed in a glovebox with 0.5 mmol of the respective aryl chloride **1k-r,** 1.1 equiv of the boronic acid **2a,** Na₂CO₃ (1.0 equiv) and 5mL of Pd(OAc)₂ + P*t*-BuCy₂ solution in cyclohexane (0.005 mol%) [Pd:L = 1:4], in 0.5mL of H₂O at 100°C for 5 h. [a] The reaction was set with 10 µL of Pd(OAc)₂ + P*t-*BuCy₂ solution in cyclohexane (0.010 mol%) [Pd:L = 1:4].
- Scale-up Experiments

### 1. Bixafen precursor

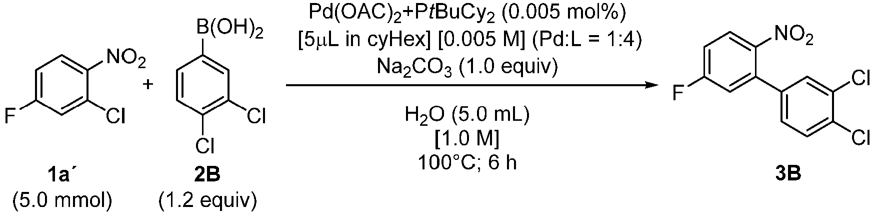

| | wt | Mw (g/mol) | mmol | eq | V |
|---|---|---|---|---|---|
| 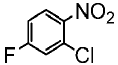 | 880mg | 175.54 | 5.0 | 1 | |
| 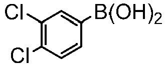 | 1.15g | 190.82 | 5.5 | 1.1 | - |
| Na₂CO₃ | 530mg | 106 | 5.0 | 1 | |
| Pd(OAc)₂+P*t*BuCy₂ | | | 0.0005 | 0.005 mol% | 50µL in CH |
| H₂O | 5.0mL | | | | |

In a glovebox, a 25.0mL pressure tube equipped with a stirrer bar was charged with 1.15g of boronic acid **2B** (1.2 equiv), 530mg of Na₂CO₃ (1.0 equiv), 50 µL of a 0.005 M solution of Pd(OAc)₂ + PtBuCy₂ (Pd:L = 1:4) in cyclohexane and 880mg of the aryl chloride **1h**. The vial was capped and transferred outside the glovebox, where 5.0mL of H₂O were added under a flow of Ar. The tube was heated at 100°C for 6 h. After this time, the tube was cooled down to rt and the mixture was diluted with 2.0mL brine and extracted with EtOAc (3 x 5.0mL), the combined organic extracts were dried over MgSO₄ and concentrate in vacuo. Subsequently, the final product was recrystallized using 10mL EtOH to obtain 1.36 g of product **3B** as yellow solid (74%). The product was characterized by ¹H and ¹³C NMR, HRMS analysis as well as elemental analysis. The results fit the previously reported data for **3B.**
• 5g-experiment Boscalid precursor

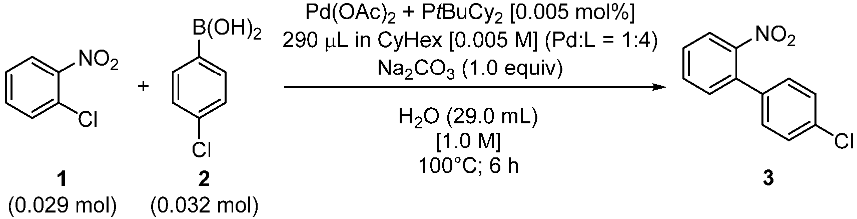

| | wt | Mw (g/mol) | mmol | eq | V |
|---|---|---|---|---|---|
| 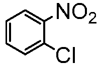 | 4.56g | 157.55 | 29 | 1 | |
| 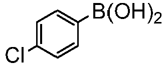 | 5.0g | 156.37 | 32 | 1.1 | - |
| Na₂CO₃ | 3.4g | 106 | 29 | 1 | |
| Pd(OAc)₂+P*t*BuCy₂ | | | 0.0005 | 0.005 mol% | 290µL in CyHex |
| H₂O | 29.0mL | | | | |

In a glovebox, a glass autoclave equipped with a spherical stirrer bar was charged with 5.0g of boronic acid **2** (1.1 equiv), 3.4g of Na₂CO₃ (1.0 equiv) and 290 µL of a 0.005 M solution of Pd(OAc)₂ + PtBuCy₂ (Pd:L = 1:4) in cyclohexane and 4.56g of the aryl chloride **1.** The autoclave was screwed inside the glovebox and transferred outside, where 29.0mL of H₂O were added under a flow of Ar. The glass autoclave was finally heated in an oil bath at 100°C for 6 h. After this time the mixture was diluted with 10.0mL of brine and subsequently extracted with EtOAc (2 x 5.0mL) and the combined organic phases were dried over MgSO₄, filtered and then analyzed by GC. Subsequently, the final product was recrystallized using 10mL EtOH to obtain 5.477g of product 3 as yellow solid (81%). The product was characterized by ¹H and ¹³C NMR, HRMS analysis as well as elemental analysis. The results fit the previously reported data for 3.

### 1. 50g-experiment Boscalid precursor

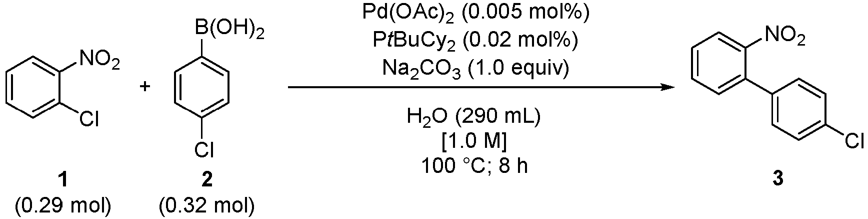

| | wt | Mw (g/mol) | mmol | eq | V |
|---|---|---|---|---|---|
| 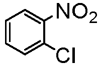 | 46g | 157.55 | 290 | 1 | |
| 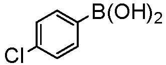 | 50g | 156.37 | 320 | 1.1 | - |
| Na₂CO₃ | 30.7g | 106 | 290 | 1 | |
| Pd(OAc)₂ | 4.0mg | 224.51 | 1.78·10⁻⁵ | 0.005 mol% | |
| P*t*-BuCy₂ | 16.0mg | 254.39 | 6.29·10⁻⁵ | 0.02 mol% | |
| H₂O | 290mL | | | | |

A 1-L Schlenk flask equipped with a spherical stirrer bar was charged with 50g of boronic acid **2** (1.1 equiv), 30.7g of Na₂CO₃ (1.0 equiv) and and 46 g of the aryl chloride **1.** The flask was transferred into a glovebox, where 4.0mg of Pd(OAc)₂ (1.78·10⁻⁵, 0.005 mol%) and 16.0mg of the ligand PtBuCy₂ (6.29·10⁻⁵, 0.02 mol%, Pd:L = 1:4) were added into the solid aryl chloride **1.** The flask was then transferred outside, where 290mL of H₂O were added under a flow of Ar. The flask was finally heated in an oil bath at 100 °C for 8 h. After this time the mixture was diluted with 100mL of brine and subsequently extracted with EtOAc (2 x 100mL) and the combined organic phases were dried over MgSO₄, filtered and then analyzed by GC.

### GC analysis of the crude reaction mixture

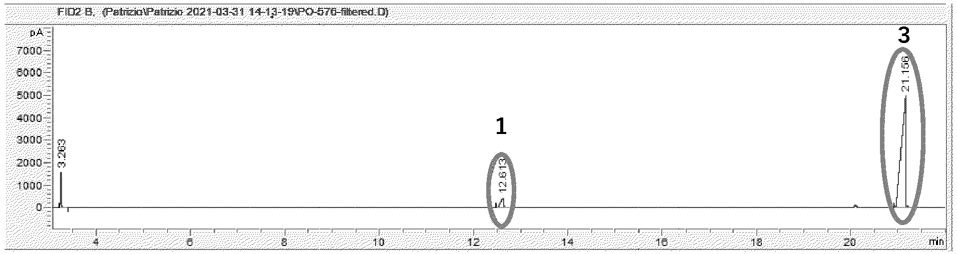

- General procedure for Boscalid three-step one-pot synthesis:

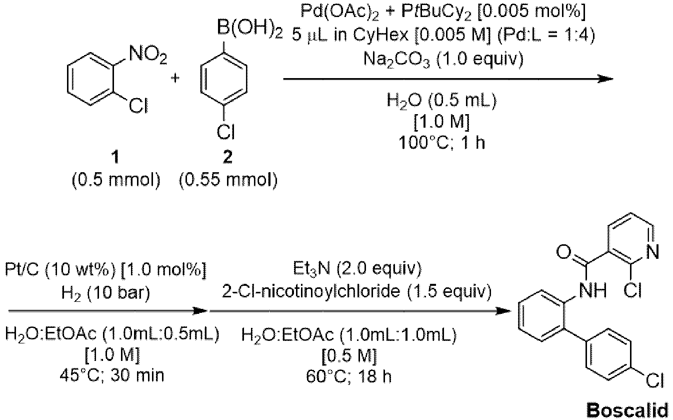

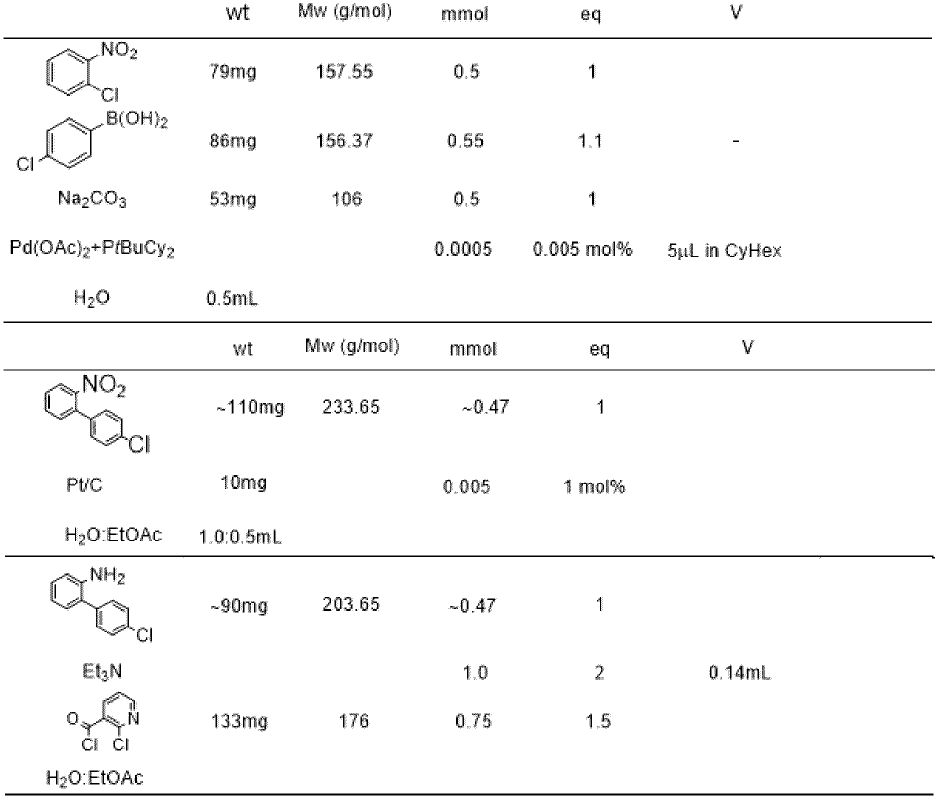

In a glovebox, a 2mL_pressure tube equipped with a spherical stirrer bar was charged with 86mg of boronic acid **2** (1.1 equiv), 53mg of Na₂CO₃ (1.0 equiv) and 5 µL of a 0.005 M solution of Pd(OAc)₂ + PtBuCy₂ (Pd:L = 1:4) in cyclohexane and 79mg of the aryl chloride **1.** The tube was capped and transferred outside the glovebox, where 0.5mL of H₂O were added under a flow of Ar. The vial was heated in an oil bath at 100°C for 1 h. After this time, the reaction mixture was cooled down to rt and was filtered through a pad of activated charcoal to remove Pd catalyst. The mixture in the pressure tube was transferred into a 10mL vial equipped with a stirrer bar and a bended needle on the septum (~110mg of product 3), filtering it through a pad of celite. The pressure tube was rinsed with 0.5mL of water and 0.5mL of EtOAc. Subsequently, the vial was charged with 10mg of Pt/C (10 wt%, 1 mol%) and the crimp cap was closed. The vial was placed in a TALL PREMEX autoclave and charged with 10 bar of H₂. The autoclave was then placed at 45°C for 30 min. After this time, the autoclave was cooled to rt and the vial was filtered over a pad of celite to remove the Pt/C catalyst. The vial was rinsed with 0.5mL of EtOAc. Subsequently, the vial was charged with 0.14mL of Et₃N (2.0 equiv) and the mixture was left stirring gently at rt for 10 min. After this time, the vial was quickly opened and 133mg of 2-chloronicotinoyl chloride (1.5 equiv) was added inside and the vial was left stirring at 60°C for 18 h. The reaction was monitored by TLC to follow the full consumption of the starting material. The mixture was the cooled down and extracted with EtOAc (3 x 2.0 mL) and brine (5.0mL). After drying it over MgSO₄ and filtering, the mixture was concentrated in vacuo. Finally, the product was purified by column chromatography (form petrol ether: ethyl acetate = 95:5 to petrol ether: ethyl acetate = 60:40) to yield 130.2 mg of white solid, characterized as **Boscalid^{®}** by¹H and ¹³C NMR spectroscopy, X-Ray analysis as well as HRMS analysis. The results fit the previously reported data for **Boscalid^{®}.**
- General procedure for Xemium three-step one-pot synthesis:

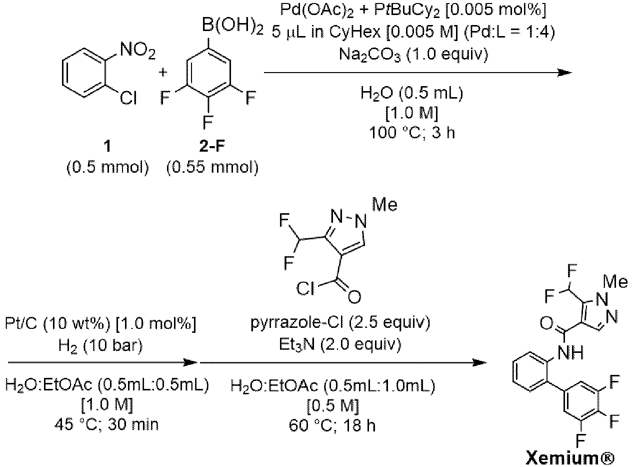

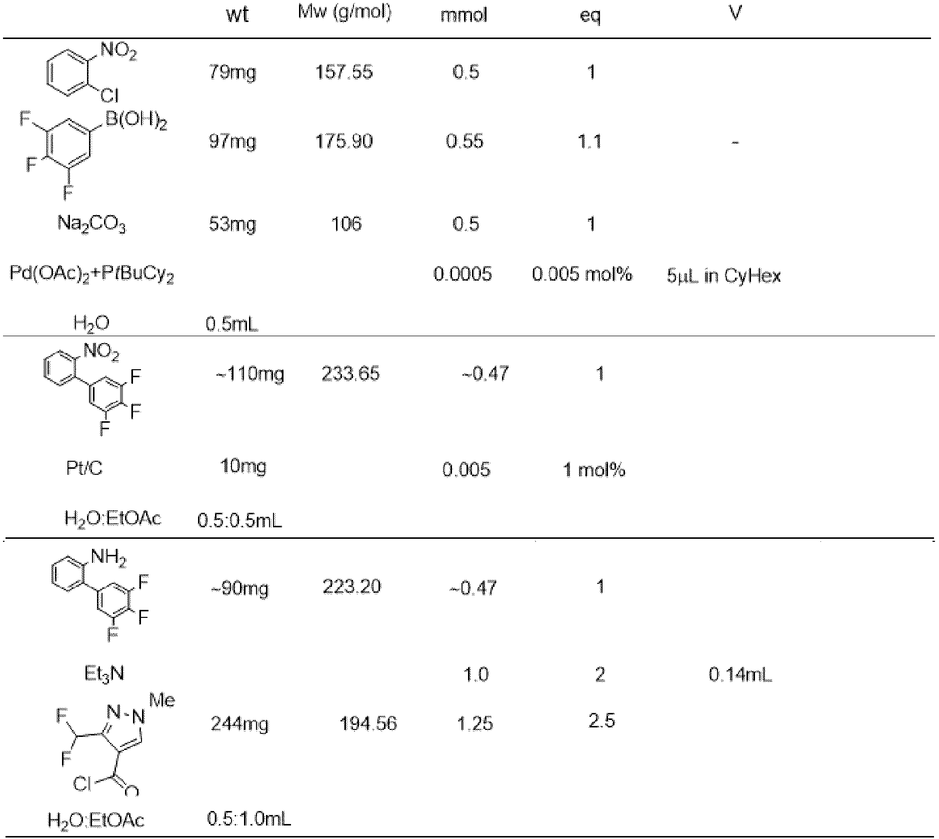

In a glovebox, a 2mL_pressure tube equipped with a spherical stirrer bar was charged with 97mg of boronic acid **2-F** (1.1 equiv), 53mg of Na₂CO₃ (1.0 equiv) and 5 µL of a 0.005 M solution of Pd(OAc)₂ + PtBuCy₂ (Pd:L = 1:4) in cyclohexane and 79mg of the aryl chloride **1.** The tube was capped and transferred outside the glovebox, where 0.5mL of H₂O were added under a flow of Ar. The vial was heated in an oil bath at 100°C for 3 h. The mixture in the pressure tube was transferred into a 10mL vial equipped with a stirrer bar and a bended needle on the septum (~110mg of product **3-F).** The pressure tube was rinsed with 0.5mL of water and 0.5mL of EtOAc. Subsequently, the vial was charged with 10mg of Pt/C (10 wt%, 1 mol%) and the crimp cap was closed. The vial was placed in a HEL CAT 7 autoclave and charged with 10 bar of H₂. The autoclave was then placed at 45°C for 30 min. After this time, the autoclave was cooled to rt and the vial was filtered over a pad of celite to remove the Pt/C catalyst. The vial was rinsed with 0.5mL of EtOAc. Subsequently, the vial was charged with 0.14mL of Et₃N (2.0 equiv) and the mixture was left stirring gently at rt for 10 min. After this time, the vial was quickly opened and 107mg of PO-539 (1.1 equiv) was added inside and the vial was left stirring at 60°C. The reaction was monitored by TLC to follow the full consumption of the starting material. The mixture was the cooled down and extracted with EtOAc (3 x 1.0 mL). After drying it over MgSO₄ and filtering, the mixture was concentrated in vacuo. Finally, the product was purified by column chromatography (form petrol ether: ethyl acetate = 95:5 to petrol ether: ethyl acetate = 60:40) to yield 126 mg of white solid, characterized as **Xemium^{®}** by ¹H and ¹³C NMR spectroscopy, X-Ray analysis as well as HRMS analysis. The results fit the previously reported data for **Xemium^{®}.**

### Comparative Experiments using Aryl-substituted Phosphine ligands:

- Investigation on aryl substituted phosphines:
   ➢ using Pd(OAc)₂ + PPh₃ (0.005 mol%): 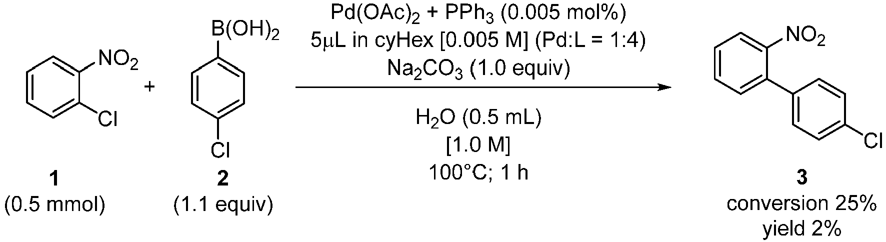
   ➢ using Pd(OAc)₂ + PCy₂Ph (0.005 mol%): 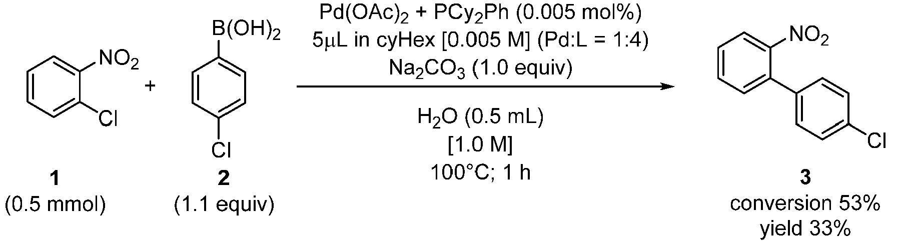
   ➢ using Pd(OAc)₂ + PCyPh₂ (0.005 mol%): 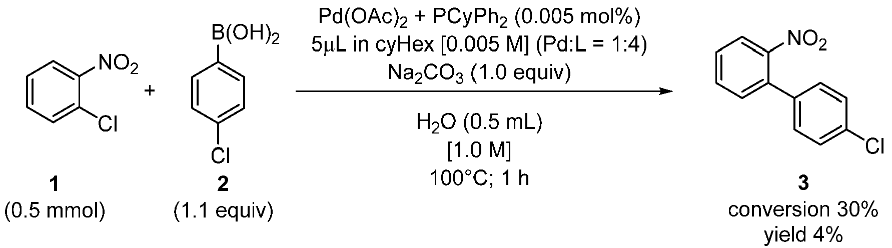
   ➢ using Pd(OAc)₂ + SPhos (0.005 mol%) [Buchwald ligand]:

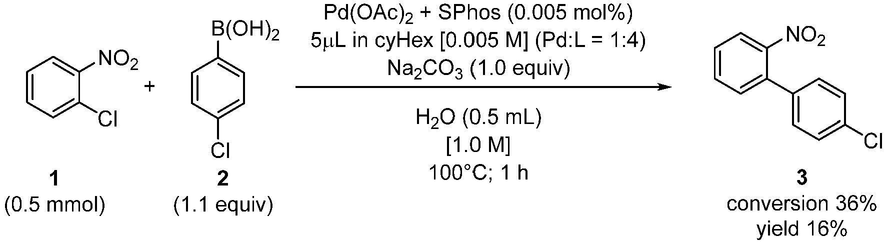

## Claims

1. A process for preparing substituted biphenyls of the formula I 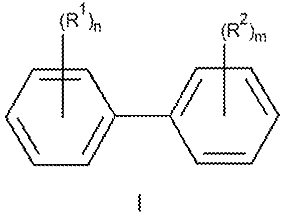 in which the substituents are each defined as follows:
R1 is cyano, nitro, F, Cl, C1-C4-alkyl, C1-C4-haloalkyl, C3-C10-cycloalkyl which may carry 1, 2, 3 or 4 C1-C4-alkyl substituents; C3-C10-halocycloalkyl, C1-C6-alkoxy, C1-C6-haloalkoxy, C1-C6-alkylcarbonyl, C1-C6-haloalkylcarbonyl, C1-C6-alkoxycarbonyl, or C1-C6-haloalkoxycarbonyl;
R2 is cyano, nitro, F, Cl, C1-C4-alkyl, C1-C4-haloalkyl, C3-C10-cycloalkyl which may carry 1, 2, 3 or 4 C1-C4-alkyl substituents; C3-C10-halocycloalkyl, C1-C6-alkoxy, C1-C6-haloalkoxy, C1-C6-alkylcarbonyl, C1-C6-haloalkylcarbonyl, C1-C6-alkoxycarbonyl, or C1-C6-haloalkoxycarbonyl; and
n is 0, 1,2 or 3, where, in case that n is 2 or 3, the R1 radicals may have identical or different definitions; and
m is 0, 1, 2 or 3, where, in case that n is 2 or 3, the R2 radicals may have identical or different definitions
which comprises reacting a compound of the formula II 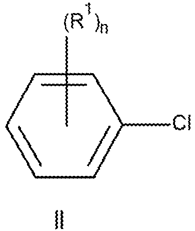
in which R1 and n is as defined as above,
in the presence of a base and of a palladium catalyst, where the palladium catalyst is introduced into the reaction in the form of
- a palladium source and a phosphorus ligand of the formula III or a salt thereof 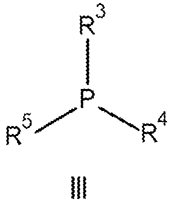 in which
R3 is C3-C18-alkyl or C3-C10-cycloalkyl which is connected to the phosphor atom at a secondary or tertiary carbon atom of the C3-C18-alkyl or C3-C10-cycloalkyl substituent
R4 is C1-C18-alkyl or C3-C10-cycloalkyl
and R5 is C1-C18-alkyl or C3-C10-cycloalkyl or
- a palladium complex containing at least one phosphorus ligand of the formula III as defined above or a salt thereof;
with water as solvent, wherein the total solvent comprises at least 95wt.-% water based on the total solvent without the use of an additional surfactant or phase transfer catalyst
with an organoboron compound of the formula IV 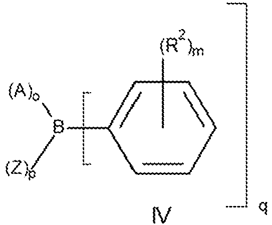
wherein R2 and m are as defined as above and the compound of formula IV is selected from the group consisting of
(i) boronic acids with o = 0, p = 2; q = 1 and Z = hydroxyl groups, or their trimers;
(ii) boronic acid derivates with o = 0, p = 2; q = 1 and Z = halogen; C1-C4-alkyl, C1-C6-alkoxy or C6-C10-aryloxy;
(iii) boronic acids or boronic acid derivatives with o = 0, p = 1; q = 2 and Z = hydroxy, halogen, C1-C4-alkyl, C1-C6-alkoxy or C6-C10-aryloxy;
(iv) mixed boronic acids or boronic acid derivatives with o =1, p = 1; q=1, A = C1-C4-alkyl and Z = hydroxy, halogen, C1-C4-alkyl, C1-C6-alkoxy or C6-C10-aryloxy;
(v) cyclic boronic esters with o = 0, p = 2 and q = 1, wherein the two Z groups form together a bridging group -O-(CH2)r-O-, wherein r is 2 or 3, so that the two Z groups, together with the boron atom to which they are attached, form a 5- or 6-membered ring, where the CH2 groups are optionally substituted by one or two C1-C4-alkyl groups;
(vi) boronates with o = 0, p = 3, q = 1 and Z = hydroxyl, halogen, C1-C4-alkyl, C1-C6-alkoxy or C6-C10-aryloxy, and accompanied by a cation which compensates the negative charge of the boronate anion;
(vii) triarylboranes with o = 0, p = 0 and q = 3;
(viii) tetraarylborates with o = 0, p = 0 and q = 4, and accompanied by a cation which compensates the negative charge of the borate anion;
where the reaction is carried out at a temperature of from 60 to 150°C.

2. A process according to claim 1, wherein the palladium catalyst is 0.01 to 0.001 mol% relating to the compound of formula II.

3. A process according to any of claims 1 to 2, wherein the solvent comprises less than 5wt.-% of organic solvents, which are only partially or not miscible with water, based on the total solvent.

4. A process according to any of claims 1 to 2, wherein the total solvent comprises at least 98wt.-% water based on the total solvent.

5. A process according to any of claims 1 to 4, wherein the solvent comprises less than 2wt.-% of organic solvents, which are only partially or not miscible with water, based on the total solvent.

6. A process according to any of claims 1 to 5, wherein the solvent comprises no other solvent miscible with water.

7. A process according to any of claims 1 to 6, wherein no additional organic solvent is present.

8. A process according to any of claims 1 to 7, wherein in formula III R3 and R4 = Cyclohexyl and R5= tertbutyl.

9. A process according to any of claims 1 to 8, wherein in formula I R1 = nitro, n=1, R2 =Cl and m=1.

10. A process according to any of claims 1 to 9, wherein formula I is 2-nitro-4'-Cl-biphenyl

11. A process according to any of claims 1 to 10, wherein in formula IV o = 0, Z = OH, p = 1, R² = para-chloro, m = 1 and q = 2.

## Patentansprüche

1. Ein Verfahren zur Herstellung substituierter Biphenyle der Formel I 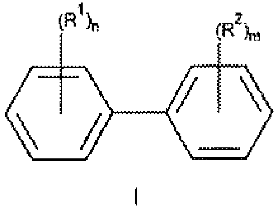 worin die Substituenten jeweils wie folgt definiert sind:
R1 ist Cyano, Nitro, F, Cl, C1-C4-Alkyl, C1-C4-Halogenalkyl, C3-C10-Cycloalkyl, das 1, 2, 3 oder 4 C1-C4-Alkylsubstituenten tragen kann; C3-C10-Halogencycloalkyl, C1-C6-Alkoxy, C1-C6-Halogenalkoxy, C1-C6-Alkylcarbonyl, C1-C6-Halogenalkylcarbonyl, C1-C6-Alkoxycarbonyl oder C1-C6-Halogenalkoxycarbonyl;
R2 ist Cyano, Nitro, F, Cl, C1-C4-Alkyl, C1-C4-Halogenalkyl, C3-C10-Cycloalkyl, das 1, 2, 3 oder 4 C1-C4-Alkylsubstituenten tragen kann; C3-C10-Halogencycloalkyl, C1-C6-Alkoxy, C1-C6-Halogenalkoxy, C1-C6-Alkylcarbonyl, C1-C6-Halogenalkylcarbonyl, C1-C6-Alkoxycarbonyl oder C1-C6-Halogenalkoxycarbonyl; und
n ist 0, 1, 2 oder 3, wobei im Fall, dass n 2 oder 3 ist, die R1-Reste gleiche oder verschiedene Definitionen haben können; und
m ist 0, 1, 2 oder 3, wobei im Fall, dass n 2 oder 3 ist, die R2-Reste gleiche oder verschiedene Definitionen haben können,
welches das Umsetzen einer Verbindung der Formel II umfasst 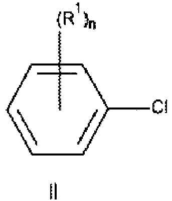
worin R1 und n wie oben definiert sind,
in Gegenwart einer Base und eines Palladiumkatalysators, wobei der Palladiumkatalysator in die Reaktion in Form von
- einer Palladiumquelle und einem Phosphorliganden der Formel III oder einem Salz davon eingebracht wird 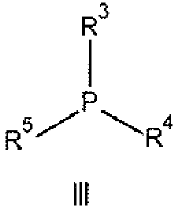 worin
R3 C3-C18-Alkyl oder C3-C10-Cycloalkyl ist, das über ein sekundäres oder tertiäres Kohlenstoffatom des C3-C18-Alkyl- oder C3-C10-Cycloalkylsubstituenten mit dem Phosphoratom verbunden ist
R4 C1-C18-Alkyl oder C3-C10-Cycloalkyl ist
und R5 C1-C18-Alkyl oder C3-C10-Cycloalkyl ist, oder
- eines Palladiumkomplexes, der mindestens einen Phosphorliganden der Formel III wie oben definiert enthält, oder eines Salzes davon;
mit Wasser als Lösungsmittel, wobei das Gesamtlösungsmittel mindestens 95 Gew.-% Wasser bezogen auf das Gesamtlösungsmittel umfasst, ohne Verwendung eines zusätzlichen Tensids oder Phasentransferkatalysators
mit einer Organoborverbindung der Formel IV 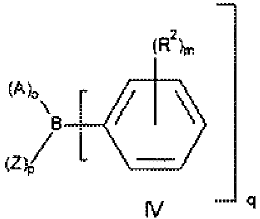
wobei R2 und m wie oben definiert sind und die Verbindung der Formel IV ausgewählt ist aus der Gruppe bestehend aus
(i) Boronsäuren mit o = 0, p = 2; q = 1 und Z = Hydroxylgruppen oder deren Trimere;
(ii) Boronsäurederivaten mit o = 0, p = 2; q = 1 und Z = Halogen; C1-C4-Alkyl, C1-C6-Alkoxy oder C6-C10-Aryloxy;
(iii) Boronsäuren oder Boronsäurederivaten mit o = 0, p = 1; q = 2 und Z = Hydroxy, Halogen, C1-C4-Alkyl, C1-C6-Alkoxy oder C6-C10-Aryloxy;
(iv) gemischten Boronsäuren oder Boronsäurederivaten mit o = 1, p = 1; q = 1, A = C1-C4-Alkyl und Z = Hydroxy, Halogen, C1-C4-Alkyl, C1-C6-Alkoxy oder C6-C10-Aryloxy;
(v) cyclischen Boronsäureestern mit o = 0, p = 2 und q = 1, wobei die beiden Z-Gruppen zusammen eine verbrückende Gruppe -O-(CH2)r-O- bilden, wobei r 2 oder 3 ist, sodass die beiden Z-Gruppen zusammen mit dem Boratom, an das sie gebunden sind, einen 5- oder 6-gliedrigen Ring bilden, wobei die CH2-Gruppen gegebenenfalls durch eine oder zwei C1-C4-Alkylgruppen substituiert sind;
(vi) Boronaten mit o = 0, p = 3, q = 1 und Z = Hydroxyl, Halogen, C1-C4-Alkyl, C1-C6-Alkoxy oder C6-C10-Aryloxy, begleitet von einem Kation, das die negative Ladung des Boronatanions kompensiert;
(vii) Triarylboranen mit o = 0, p = 0 und q = 3;
(viii) Tetraarylboraten mit o = 0, p = 0 und q = 4, begleitet von einem Kation, das die negative Ladung des Boratanions kompensiert;
wobei die Reaktion bei einer Temperatur von 60 bis 150 ° C durchgeführt wird.

2. Ein Verfahren gemäß Anspruch 1, wobei der Palladiumkatalysator 0,01 bis 0,001 mol% bezogen auf die Verbindung der Formel II beträgt.

3. Ein Verfahren gemäß einem der Ansprüche 1 bis 2, wobei das Lösungsmittel weniger als 5 Gew.-% organische Lösungsmittel, die nur teilweise oder nicht mit Wasser mischbar sind, bezogen auf das Gesamtlösungsmittel umfasst.

4. Ein Verfahren gemäß einem der Ansprüche 1 bis 2, wobei das Gesamtlösungsmittel mindestens 98 Gew.-% Wasser bezogen auf das Gesamtlösungsmittel umfasst.

5. Ein Verfahren gemäß einem der Ansprüche 1 bis 4, wobei das Lösungsmittel weniger als 2 Gew.-% organische Lösungsmittel, die nur teilweise oder nicht mit Wasser mischbar sind, bezogen auf das Gesamtlösungsmittel umfasst.

6. Ein Verfahren gemäß einem der Ansprüche 1 bis 5, wobei das Lösungsmittel kein weiteres mit Wasser mischbares Lösungsmittel enthält.

7. Ein Verfahren gemäß einem der Ansprüche 1 bis 6, wobei kein zusätzliches organisches Lösungsmittel vorhanden ist.

8. Ein Verfahren gemäß einem der Ansprüche 1 bis 7, wobei in Formel III R3 und R4 = Cyclohexyl und R5 = Tertbutyl sind.

9. Ein Verfahren gemäß einem der Ansprüche 1 bis 8, wobei in Formel I R1 = Nitro, n = 1, R2 = CI und m = 1 sind.

10. Ein Verfahren gemäß einem der Ansprüche 1 bis 9, wobei Formel I 2-Nitro-4'-Cl-biphenyl ist.

11. Ein Verfahren gemäß einem der Ansprüche 1 bis 10, wobei in Formel IV o = 0, Z = OH, p = 1, R² = para-Chlor, m = 1 und q = 2 sind.

## Revendications

1. Un procédé de préparation de biphényles substitués de la formule I 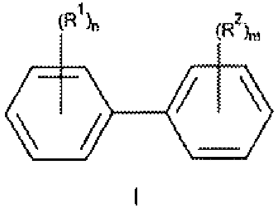 dans laquelle les substituants sont chacun définis comme suit :
R1 est cyano, nitro, F, Cl, C1-C4-alkyle, C1-C4-haloalkyle, C3-C10-cycloalkyle pouvant porter 1, 2, 3 ou 4 substituants C1-C4-alkyle ; C3-C10-halocycloalkyle, C1-C6-alcoxy, C1-C6-haloalcoxy, C1-C6-alkylcarbonyle, C1-C6-haloalkylcarbonyle, C1-C6-alcoxycarbonyle, ou C1-C6-haloal-coxycarbonyle ;
R2 est cyano, nitro, F, Cl, C1-C4-alkyle, C1-C4-haloalkyle, C3-C10-cycloalkyle pouvant porter 1, 2, 3 ou 4 substituants C1-C4-alkyle ; C3-C10-halocycloalkyle, C1-C6-alcoxy, C1-C6-haloalcoxy, C1-C6-alkylcarbonyle, C1-C6-haloalkylcarbonyle, C1-C6-alcoxycarbonyle, ou C1-C6-haloal-coxycarbonyle ; et
n est 0, 1, 2 ou 3, où, dans le cas où n est 2 ou 3, les radicaux R1 peuvent avoir des définitions identiques ou différentes ; et
m est 0, 1, 2 ou 3, où, dans le cas où n est 2 ou 3, les radicaux R2 peuvent avoir des définitions identiques ou différentes
qui comprend la réaction d'un composé de la formule II 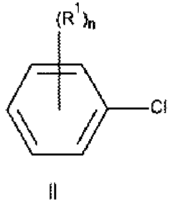
dans laquelle R1 et n sont tels que définis ci-dessus,
en présence d'une base et d'un catalyseur au palladium, où le catalyseur au palladium est introduit dans la réaction sous la forme de
- une source de palladium et un ligand phosphoré de la formule III ou un sel de celui-ci 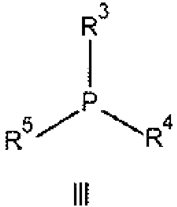 dans laquelle
R3 est C3-C18-alkyle ou C3-C10-cycloalkyle qui est relié à l'atome de phosphore en un carbone secondaire ou tertiaire du substituant C3-C18-alkyle ou C3-C10-cycloalkyle
R4 est C1-C18-alkyle ou C3-C10-cycloalkyle
et R5 est C1-C18-alkyle ou C3-C10-cycloalkyle ou
- un complexe de palladium contenant au moins un ligand phosphoré de la formule III tel que défini ci-dessus ou un sel de celui-ci ;
avec de l'eau comme solvant, où le solvant total comprend au moins 95% en poids d'eau par rapport au solvant total sans utilisation d'un tensioactif supplémentaire ou d'un catalyseur de transfert de phase
avec un composé organoboré de la formule IV 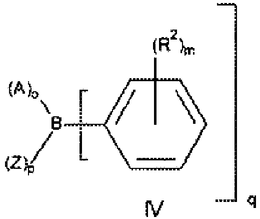
dans laquelle R2 et m sont tels que définis ci-dessus et le composé de formule IV est sélectionné dans le groupe constitué de
(i) acides boroniques avec o = 0, p = 2 ; q = 1 et Z = groupes hydroxyle, ou leurs trimères ;
(ii) dérivés d'acides boroniques avec o = 0, p = 2 ; q = 1 et Z = halogène ; C1-C4-alkyle, C1-C6-alcoxy ou C6-C10-aryloxy ;
(iii) acides boroniques ou dérivés d'acides boroniques avec o = 0, p = 1 ; q = 2 et Z = hydroxy, halogène, C1-C4-alkyle, C1-C6-alcoxy ou C6-C10-aryloxy ;
(iv) acides boroniques mixtes ou dérivés d'acides boroniques mixtes avec o = 1, p = 1 ; q = 1, A = C1-C4-alkyle et Z = hydroxy, halogène, C1-C4-alkyle, C1-C6-alcoxy ou C6-C10-aryloxy ;
(v) esters boroniques cycliques avec o = 0, p = 2 et q = 1, dans lesquels les deux groupes Z forment ensemble un groupe pontant -O-(CH2)r-O-, où r est 2 ou 3, de sorte que les deux groupes Z, ensemble avec l'atome de bore auquel ils sont attachés, forment un cycle à 5 ou 6 chaînons, où les groupes CH2 sont optionnellement substitués par un ou deux groupes C1-C4-alkyle ;
(vi) boronates avec o = 0, p = 3, q = 1 et Z = hydroxyle, halogène, C1-C4-alkyle, C1-C6-alcoxy ou C6-C10-aryloxy, et accompagnés d'un cation qui compense la charge négative de l'anion boronate ;
(vii) triarylboranes avec o = 0, p = 0 et q = 3 ;
(viii) tétraarylborates avec o = 0, p = 0 et q = 4, et accompagnés d'un cation qui compense la charge négative de l'anion borate ;
où la réaction est effectuée à une température de 60 à 150 ° C.

2. Un procédé selon la revendication 1, dans lequel le catalyseur au palladium est de 0,01 à 0,001 mol% par rapport au composé de formule II.

3. Un procédé selon l'une quelconque des revendications 1 à 2, dans lequel le solvant comprend moins de 5% en poids de solvants organiques, qui sont seulement partiellement ou non miscibles avec l'eau, par rapport au solvant total.

4. Un procédé selon l'une quelconque des revendications 1 à 2, dans lequel le solvant total comprend au moins 98% en poids d'eau par rapport au solvant total.

5. Un procédé selon l'une quelconque des revendications 1 à 4, dans lequel le solvant comprend moins de 2% en poids de solvants organiques, qui sont seulement partiellement ou non miscibles avec l'eau, par rapport au solvant total.

6. Un procédé selon l'une quelconque des revendications 1 à 5, dans lequel le solvant ne comprend aucun autre solvant miscible avec l'eau.

7. Un procédé selon l'une quelconque des revendications 1 à 6, dans lequel aucun solvant organique supplémentaire n'est présent.

8. Un procédé selon l'une quelconque des revendications 1 à 7, dans lequel dans la formule III R3 et R4 = cyclohexyle et R5 = tertbutyle.

9. Un procédé selon l'une quelconque des revendications 1 à 8, dans lequel dans la formule I R1 = nitro, n = 1, R2 = CI et m = 1.

10. Un procédé selon l'une quelconque des revendications 1 à 9, dans lequel la formule I est le 2-nitro-4'-Cl-biphényle.

11. Un procédé selon l'une quelconque des revendications 1 à 10, dans lequel dans la formule IV o = 0, Z = OH, p = 1, R² = para-chloro, m = 1 et q = 2.
